# EUROPEAN PATENT SPECIFICATION

(11) **EP 3 419 999 B1**
(45) Date of publication and mention of the grant of the patent: **04.08.2021**
(21) Application number: 17707530.6
(22) Date of filing: 24.02.2017
(51) Int. Cl.: C07K 16/28

(54) **ANTIBODIES HAVING SPECIFICITY FOR BTLA AND USES THEREOF**
ANTIKÖRPER MIT SPEZIFIZITÄT FÜR BTLA UND VERWENDUNGEN DAVON
ANTICORPS AYANT UNE SPÉCIFICITÉ POUR LE BTLA ET LEURS UTILISATIONS

(30) Priority: 26.02.2016 EP 16305223
(43) Date of publication of application: 02.01.2019
(73) Proprietor: (INSERM) Institut National de la Santé et de la Recherche Médicale, 75013 Paris (FR); Centre National de la Recherche Scientifique (CNRS), 75016 Paris (FR); Université d'Aix Marseille, 13284 Marseille Cedex 07 (FR); Institut Jean Paoli & Irène Calmettes, 13009 Marseille (FR)
(72) Inventor: OLIVE, Daniel, 13009 Marseille (FR)
(74) Representative: Plasseraud IP
(86) International application number: PCT/EP2017/054342
(87) International publication number: WO 2017/144668

(56) References cited:
- WO-A1-2011/014438
- WO-A1-2014/183885
- J. GERTNER-DARDENNE ET AL: "The co-receptor BTLA negatively regulates human V 9V 2 T-cell proliferation: a potential way of immune escape for lymphoma cells", BLOOD, vol. 122, no. 6, 8 August 2013 (2013-08-08), pages 922-931, XP055146739, ISSN: 0006-4971, DOI: 10.1182/blood-2012-11-464685
- Perez De La Lastra ET AL: "Epitope mapping of 10 monoclonal antibodies against the pig analogue of human membrane cofactor protein (MCP)", Immunology, vol. 96, no. 4, 1 April 1999 (1999-04-01), pages 663-670, XP55572134, GB ISSN: 0019-2805, DOI: 10.1046/j.1365-2567.1999.00732.x

### SEQUENCE LISTING

<110> Inserm
<120> ANTIBODIES HAVING SPECIFICITY FOR BTLA AND USES THEREOF
<130> BIO15432 OLIVE / MC
<160> 7
<170> PatentIn version 3.5
<210> 1
   <211> 120
   <212> PRT
   <213> Artificial Sequence
<220>
   <223> VH sequence of 629.3
<400> 1
   Gln Val Gln Leu Arg Glu Ser Gly Pro Gly Leu Val Ala Pro Ser Gln
   1               5                   10                  15
   Ser Leu Ser Ile Thr Cys Thr Val Ser Gly Phe Ser Leu Asn Thr Tyr
               20                  25                  30
   Gly Val Ser Trp Val Arg Gln Pro Pro Gly Lys Gly Leu Glu Trp Leu
           35                  40                  45
   Gly Val Val Trp Gly Asp Gly Asn Thr Asn Tyr His Ser Val Val Ile
       50                  55                  60
   Ser Arg Leu Thr Ile Asn Lys Asp Asn Ser Lys Ser Gln Val Phe Leu
   65                  70                  75                  80
   Lys Leu Asn Ser Leu Gln Thr Asp Asp Thr Ala Thr Tyr Tyr Cys Ala
                   85                  90                  95
   Lys Cys Tyr Gly Ser Arg Phe Asp Tyr Ala Met Asp Tyr Trp Gly Gln
               100                 105                 110
   Gly Thr Ser Val Thr Val Ser Ser
           115                 120
<210> 2
   <211> 112
   <212> PRT
   <213> Artificial Sequence
<220>
   <223> VL sequence of 629.3
<400> 2
   Asp Ile Val Met Ser Gln Ser Pro Ser Ser Leu Ala Val Ser Ile Gly
   1               5                   10                  15
   Glu Gln Val Thr Met Ser Cys Lys Ser Ser Gln Ser Leu Leu Ser Ser
               20                  25                  30
   Arg Thr Arg Lys Asn Tyr Leu Ala Trp Tyr Gln Gln Arg Pro Gly Gln
           35                  40                  45
   Ser Pro Lys Leu Leu Ile Tyr Trp Ala Ser Thr Arg Glu Ser Gly Val
       50                  55                  60
   Pro Asp Arg Phe Thr Gly Ser Gly Ser Gly Thr Asp Phe Thr Leu Thr
   65                  70                  75                  80
   Ile Ser Ser Val Gln Ala Glu Asp Leu Ala Val Tyr Tyr Cys Lys Gln
                   85                  90                  95
   Ser Tyr Asn Leu Pro Thr Phe Gly Gly Gly Thr Arg Leu Glu Ile Lys
               100                 105                 110
<210> 3
   <211> 360
   <212> DNA
   <213> Artificial Sequence
<220>
   <223> DNA VH Region of the 629.3 mAb
<400> 3
   caggtgcagc tgagggagtc aggacctggc ctggtggcgc cctcacagag cctgtccatc      60
   acgtgcactg tctcagggtt ctcattaaac acctatggtg taagttgggt tcgccagcct     120
   ccaggaaagg gtctggagtg gctgggagta gtatggggtg acgggaacac aaattatcat     180
   tcagttgtca tatccagact gaccatcaat aaggataatt ccaagagcca agttttctta     240
   aaactgaaca gtctgcaaac tgatgacaca gccacgtact actgtgccaa atgctacggt     300
   agtaggttcg actacgctat ggactactgg ggtcaaggaa cctcagtcac cgtctcctca     360
<210> 4
   <211> 336
   <212> DNA
   <213> Artificial Sequence
<220>
   <223> DNA VL region of the 629.3 mAb
<400> 4
   gacattgtga tgtcacagtc tccatcttcc ctggctgtgt caataggaga gcaggtcact       60
   atgagctgca aatccagtca gagtctgctc agcagtagaa cccgaaagaa ctacttggct      120
   tggtaccagc agagaccagg acagtctcct aaactgctga tctactgggc atccactcgg      180
   gaatctgggg tccctgatcg cttcacaggc agtggatctg ggacagattt cactctcacc      240
   atcagcagtg tgcaggctga agacctggca gtttattact gcaagcaatc ttataatctt      300
   ccgacgttcg gtggaggcac caggctggaa atcaaa                                336
<210> 5
   <211> 15
   <212> PRT
   <213> Artificial Sequence
<220>
   <223> SEQ ID NO:5
<400> 5
   Val Lys Leu Glu Asp Arg Gln Thr Ser Trp Lys Glu Glu Lys Asn
   1               5                   10                  15
<210> 6
   <211> 13
   <212> PRT
   <213> Artificial Sequence
<220>
   <223> SEQ ID NO:6
<400> 6
   Pro Ser Lys Asp Glu Met Ala Ser Arg Pro Trp Leu Leu
   1               5                   10
<210> 7
   <211> 289
   <212> PRT
   <213> Artificial Sequence
<220>
   <223> SEQ ID NO:7
<400> 7
   Met Lys Thr Leu Pro Ala Met Leu Gly Thr Gly Lys Leu Phe Trp Val
   1               5                   10                  15
   Phe Phe Leu Ile Pro Tyr Leu Asp Ile Trp Asn Ile His Gly Lys Glu
               20                  25                  30
   Ser Cys Asp Val Gln Leu Tyr Ile Lys Arg Gln Ser Glu His Ser Ile
           35                  40                  45
   Leu Ala Gly Asp Pro Phe Glu Leu Glu Cys Pro Val Lys Tyr Cys Ala
       50                  55                  60
   Asn Arg Pro His Val Thr Trp Cys Lys Leu Asn Gly Thr Thr Cys Val
   65                  70                  75                  80
   Lys Leu Glu Asp Arg Gln Thr Ser Trp Lys Glu Glu Lys Asn Ile Ser
                   85                  90                  95
   Phe Phe Ile Leu His Phe Glu Pro Val Leu Pro Asn Asp Asn Gly Ser
               100                 105                 110
   Tyr Arg Cys Ser Ala Asn Phe Gln Ser Asn Leu Ile Glu Ser His Ser
           115                 120                 125
   Thr Thr Leu Tyr Val Thr Asp Val Lys Ser Ala Ser Glu Arg Pro Ser
       130                 135                 140
   Lys Asp Glu Met Ala Ser Arg Pro Trp Leu Leu Tyr Arg Leu Leu Pro
   145                 150                 155                 160
   Leu Gly Gly Leu Pro Leu Leu Ile Thr Thr Cys Phe Cys Leu Phe Cys
                   165                 170                 175
   Cys Leu Arg Arg His Gln Gly Lys Gln Asn Glu Leu Ser Asp Thr Ala
               180                 185                 190
   Gly Arg Glu Ile Asn Leu Val Asp Ala His Leu Lys Ser Glu Gln Thr
           195                 200                 205
   Glu Ala Ser Thr Arg Gln Asn Ser Gln Val Leu Leu Ser Glu Thr Gly
       210                 215                 220
   Ile Tyr Asp Asn Asp Pro Asp Leu Cys Phe Arg Met Gln Glu Gly Ser
   225                 230                 235                 240
   Glu Val Tyr Ser Asn Pro Cys Leu Glu Glu Asn Lys Pro Gly Ile Val
                   245                 250                 255
   Tyr Ala Ser Leu Asn His Ser Val Ile Gly Pro Asn Ser Arg Leu Ala
               260                 265                 270
   Arg Asn Val Lys Glu Ala Pro Thr Glu Tyr Ala Ser Ile Cys Val Arg
           275                 280                 285
   Ser

## Description

### FIELD OF THE INVENTION:

The present invention relates to antibodies having specificity for BTLA and uses thereof.

### BACKGROUND OF THE INVENTION:

The importance of the antitumor immunity in the outcome and control of cancer is now recognized. Innate and adaptive immunity maintains effector cells such as lymphocytes and natural killer cells that distinguish normal cells from "modified" cells as in the case of tumor cells. However, most often tumor cells are able to evade immune recognition and destruction. The mechanisms of tumor escape are numerous, but the immunosuppressive action of coinhibitory molecules has emerged this last decade as the most attractive one for imaging new treatments of cancer. Activation of lymphocytes is indeed regulated by both costimulatory and coinhibitory molecules, belonging to the B7/CD28 superfamily (also known as the Immunoglobulin (Ig) superfamily) and the TNF/TNFR superfamily. The balance between these signals determines the lymphocyte activation and consequently regulates the immune response. These costimulatory and coinhibitory molecules were called "immune checkpoints". The two coinhibitory molecules that have been the most extensively studied and for which antagonist monoclonal antibodies (mAbs) already tested in clinical trials are cytotoxic T-lymphocyte-associated antigen 4 (CTLA4; also known as CD152) and programmed cell death protein 1 (PD1; also known as CD279). One other immune checkpoint is BTLA. BTLA (B- and T-lymphocyte attenuator, also known as CD272) is a member of the B7/CD28 superfamily and was first identified as an inhibitory receptor on T cells on the basis of the enhanced T cell responses that were observed in Btla-knockout mice. Its ligand HVEM (herpesvirus entry mediator, also known as TNFRSR14) is a member of the other cosignaling molecules family, the TNF/TNFR superfamily. HVEM has emerged as a major and complex cosignaling molecule, reflected by the network of its ligands from both the TNF/TNFR superfamily (LIGHT (lymphotoxins, inducible, competes with herpes simplex virus (HSV) glycoprotein D for HVEM, expressed by T cells) and lymphotoxin-a) and the Ig superfamily (BTLA and CD160) and the dual functions depending on the ligand engaged. Thus, HVEM provides a stimulatory signal following engagement with the TNF member LIGHT (TNFSF14) on T and B cells. In contrast, HVEM can also provide an inhibitory signal to T cells upon binding to BTLA or CD160. Thus, HVEM may be viewed as a molecular switch, capable of facilitating both stimulatory and inhibitory cosignaling in T cells. It also seems that signaling between HVEM and its ligands can be bidirectional, depending on the specific combination of interactions. Despite the complexity of ligand binding, the inhibitory function of HVEM seems to be dominant as demonstrated by HVEM-/- mice studies. BTLA is expressed by lymphoid and myeloid cells, with particularly high expression by peripheral B cells and plasmacytoid dendritic cells and lower expression by CD11c+ DCs and naive T cells. Similarly to CTLA-4, ICOS, and PD-1, BTLA is induced on CD4+ T cells during activation. Several antibodies having specificity for BTLA and inhibiting the BTLA/HVEM interaction have been described in the prior art (e.g. in WO2010106051, WO2011014438, WO2008076560, and WO2014184360).

### SUMMARY OF THE INVENTION:

The present invention relates to antibodies having specificity for BTLA and uses thereof. It is defined by the claims.

### DETAILED DESCRIPTION:

The technical information set out below may in some respects go beyond the disclosure of the invention, which is defined exclusively by the appended claims. The additional technical information is provided to place the actual invention in a broader technical context and to illustrate possible related technical developments. Such additional technical information which does not fall within the scope of the appended claims, is not part of the invention.

The inventors now characterized a new monoclonal antibody having specificity for BTLA (i.e. the 629.3 mAb) and that does not inhibit the BTLA/HVEM interaction but still surprisingly inhibits the activation of BTLA. Accordingly the antibody thus represents a new way of stimulating immune response, in particular for increasing proliferation of Vγ9Vδ2 T cells in a subject in need thereof. Moreover, as the 629.3 mAb can bind BTLA independently of HVEM, the antibody could advantageously be useful for the treatment of HVEM negative cancers by activating Vγ9Vδ2 T cells.

As used herein the term "BTLA" has its general meaning in the art and denotes an abbreviation for "B and T lymphocyte attenuator". In particular, the term BTLA refers to human BTLA having the amino acid sequence of SEQ ID NO:7.

SEQ ID NO:7: BTLA isoform 1 precursor (*Homo sapiens*):

As used herein the term "antibody" or "immunoglobulin" have the same meaning, and will be used equally in the present disclosure. The term "antibody" as used herein refers to immunoglobulin molecules and immunologically active portions of immunoglobulin molecules, i.e., molecules that contain an antigen binding site that immunospecifically binds an antigen. As such, the term antibody encompasses not only whole antibody molecules, but also antibody fragments as well as variants (including derivatives) of antibodies and antibody fragments. In natural antibodies, two heavy chains are linked to each other by disulfide bonds and each heavy chain is linked to a light chain by a disulfide bond. There are two types of light chain, lambda (1) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CHI, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from nonhypervariable or framework regions (FR) can participate to the antibody binding site or influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated L-CDR1, L-CDR2, L- CDR3 and H-CDR1, H-CDR2, H-CDR3, respectively. An antigen-binding site, therefore, typically includes six CDRs, comprising the CDR set from each of a heavy and a light chain V region. Framework Regions (FRs) refer to amino acid sequences interposed between CDRs. The residues in antibody variable domains are conventionally numbered according to a system devised by Kabat et al. This system is set forth in Kabat et al., 1987, in Sequences of Proteins of Immunological Interest, US Department of Health and Human Services, NIH, USA (hereafter "Kabat et al."). This numbering system is used in the present specification. The Kabat residue designations do not always correspond directly with the linear numbering of the amino acid residues in SEQ ID sequences. The actual linear amino acid sequence may contain fewer or additional amino acids than in the strict Kabat numbering corresponding to a shortening of, or insertion into, a structural component, whether framework or complementarity determining region (CDR), of the basic variable domain structure. The correct Kabat numbering of residues may be determined for a given antibody by alignment of residues of homology in the sequence of the antibody with a "standard" Kabat numbered sequence. The CDRs of the heavy chain variable domain are located at residues 31-35 (H-CDR1), residues 50-65 (H-CDR2) and residues 95-102 (H-CDR3) according to the Kabat numbering system. The CDRs of the light chain variable domain are located at residues 24-34 (L-CDR1), residues 50-56 (L-CDR2) and residues 89-97 (L-CDR3) according to the Kabat numbering system.

In specific aspects of the disclosure, an antibody provided herein is an antibody fragment, and more particularly any protein including an antigen-binding domain of an antibody as disclosed herein. Antibody fragments include, but are not limited to, Fv, Fab, F(ab')2, Fab', dsFv, scFv, sc(Fv)2 and diabodies. As used herein, the term "specificity" refers to the ability of an antibody to detectably bind an epitope presented on an antigen, such as a BTLA, while having relatively little detectable reactivity with non-BTLA proteins or structures (such as other proteins presented on T cells, or on other cell types). Specificity can be relatively determined by binding or competitive binding assays, using, e.g., Biacore instruments, as described elsewhere herein. Specificity can be exhibited by, e.g., an about 10:1, about 20:1, about 50:1, about 100:1, 10.000:1 or greater ratio of affinity/avidity in binding to the specific antigen versus nonspecific binding to other irrelevant molecules (in this case the specific antigen is a BTLA polypeptide). The term "affinity", as used herein, means the strength of the binding of an antibody to an epitope. The affinity of an antibody is given by the dissociation constant Kd, defined as [Ab] x [Ag] / [Ab-Ag], where [Ab-Ag] is the molar concentration of the antibody-antigen complex, [Ab] is the molar concentration of the unbound antibody and [Ag] is the molar concentration of the unbound antigen. The affinity constant Ka is defined by 1/Kd. Preferred methods for determining the affinity of mAbs can be found in Harlow, et al., Antibodies: A Laboratory Manual, Cold Spring Harbor Laboratory Press, Cold Spring Harbor, N.Y., 1988), Coligan et al., eds., Current Protocols in Immunology, Greene Publishing Assoc. and Wiley Interscience, N.Y., (1992, 1993), and Muller, Meth. Enzymol. 92:589-601 (1983), which references are entirely incorporated herein by reference. One preferred and standard method well known in the art for determining the affinity of mAbs is the use of surface plasmonic resonance (SPR) method using for example Biacore® instruments.

In one aspect, the present disclosure relates to an antibody having specificity for BTLA characterized in that
(i) it does not block the binding of HVEM to BTLA, and,
(ii) it increases the proliferation of Vγ9VδT cells.

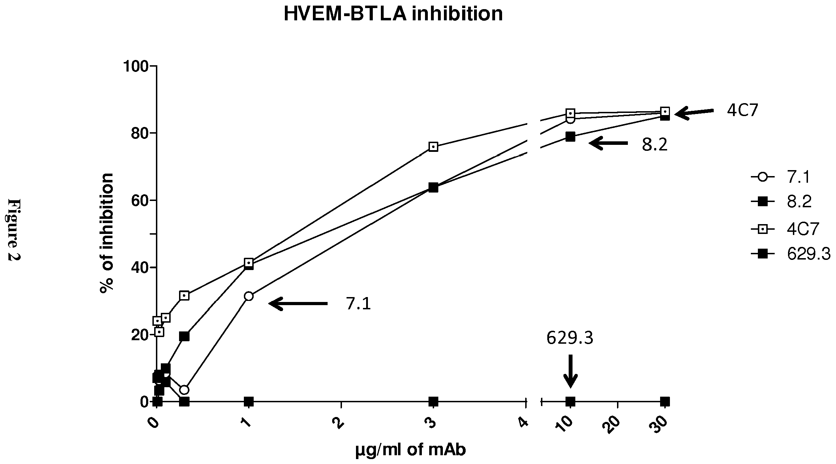

Anti-BTLA antibodies which do not block the binding of HVEM to BTLA can be screened using the cellular assay as disclosed in the Example and the legend of Figure 2. In specific aspects of the disclosure, the anti-BTLA antibodies according to the present disclosure do not block the binding of HVEM-Fc to COS cells expressing recombinant BTLA at their surface (as shown in the Example for the specific embodiment of mAb 629.3).

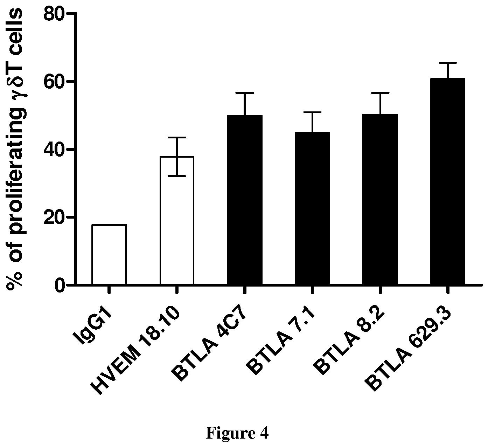

An increase of the proliferation of Vγ9VδT cells by anti-BTLA antibodies can be tested using the assay as described in the Example and the legend of Figure 4. In specific aspects of the disclosures, the anti-BTLA antibodies increase of the proliferation of Vγ9VδT cells to a level at least equal or superior to 8.2 mAb as tested using the assay described in the Example, said 8.2 mAb being disclosed in WO2010106051.

In one aspects of the disclosure, the isolated anti-BTLA antibody of the disclosure further does not compete with 4C7 mAb for binding to BTLA, said 4C7 mAb antibody being disclosed in WO2011014438.

In other aspects of the disclosure, the present disclosure relates to an antibody having specificity for BTLA characterized in that the antibody competes for binding to BTLA with the 629.3 mAb.

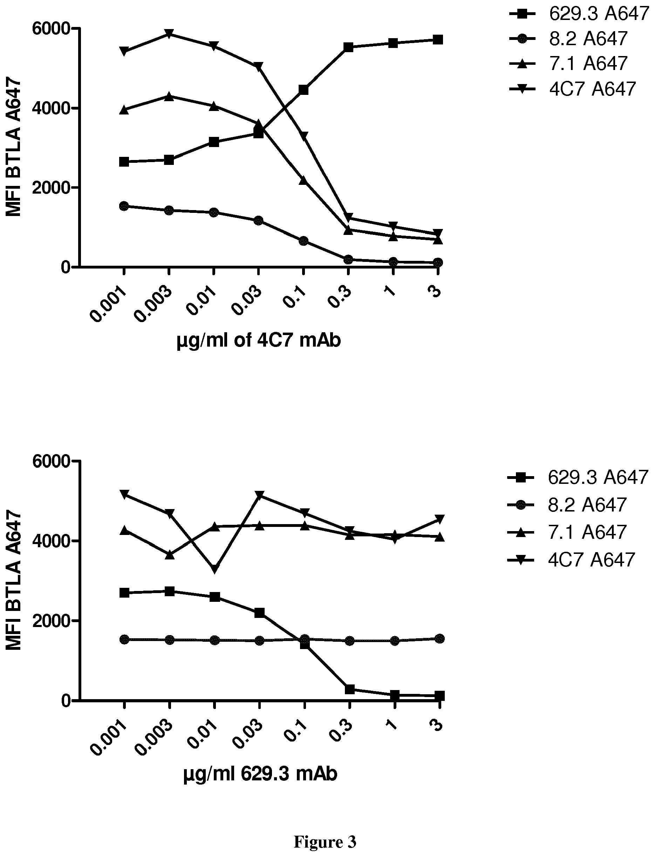

Epitope binning can be used to identify antibodies that fall within the scope of the claimed disclosure. Epitope binning refers to the use of competitive binding assays to identity pairs of antibodies that are, or are not, capable of binding BTLA simultaneously, thereby identifying pairs of antibodies that bind to the same or overlapping epitopes on BTLA. Epitope binning experiments provide evidence that antigenically distinct epitopes are present. Competition for binding can be evaluated for any pair of antibodies or fragments. For example, using the appropriate detection reagents, the binding specificity of antibodies or binding fragments from any source can be compared to the binding specificity of the monoclonal antibodies disclosed herein. Epitope binning can be performed with "isolated antibodies" or with cell culture supernatants. Frequently, binning is performed with first round clonal supernatants to guide the choice of clones to be developed further. The antibodies to be compared should be substantially homogeneous antigen binding domains. The antibodies of the present disclosure may be assayed for specific binding by any method known in the art. Many different competitive binding assay format(s) can be used for epitope binning. The immunoassays which can be used include, but are not limited to, competitive assay systems using techniques such western blots, radioimmunoassays, ELISA, "sandwich" immunoassays, immunoprecipitation assays, precipitin assays, gel diffusion precipitin assays, immunoradiometric assays, fluorescent immunoassays, protein A immunoassays, and complement-fixation assays. Such assays are routine and well known in the art (see, e.g., Ausubel et al., eds, 1994 Current Protocols in Molecular Biology, Vol. 1, John Wiley & sons, Inc., New York). For example, the BIACORE® (GE Healthcare, Piscaataway, NJ) is one of a variety of surface plasmon resonance assay formats that are routinely used to epitope bin panels of monoclonal antibodies. Additionally, routine cross-blocking assays such as those described in Antibodies, A Laboratory Manual, Cold Spring Harbor Laboratory, Ed Harlow and David Lane, 1988, can be performed. Typically, cross-competition can be determined by the method described in the EXAMPLE and Figure 3.

According to the present disclosure, the VH region of the 629.3 mAb consists of the sequence of SEQ ID NO:1. Accordingly, the H-CDR1 of the 629.3 mAb is defined by the sequence ranging from the amino acid residue at position 31 to the amino acid residue at position 35 in SEQ ID NO:1. Accordingly, the H-CDR2 of the 629.3 mAb is defined by the sequence ranging from the amino acid residue at position 50 to the amino acid residue at position 65 in SEQ ID NO:1. Accordingly, the H-CDR3 of the 629.3 mAb is defined by the sequence ranging from the amino acid residue at position 98 to the amino acid residue at position 109 in SEQ ID NO:1.

SEQ ID NO:1: VH region of the 629.3 mAb FR1-CDR1-FR2-CDR2-FR3-CDR3-FR4

According to the present disclosure, the VL region of the 629.3 mAb antibody consists of the sequence of SEQ ID NO:2. Accordingly, the L-CDR1 of the 629.3 mAb is defined by the sequence ranging from the amino acid residue at position 24 to the amino acid residue at position 40 in SEQ ID NO:2. Accordingly, the L-CDR2 of the 629.3 mAb is defined by the sequence ranging from the amino acid residue at position 56 to the amino acid residue at position 62 in SEQ ID NO:2. Accordingly, the L-CDR3 of the 629.3 mAb is defined by the sequence ranging from the amino acid residue at position 95 to the amino acid residue at position 102 in SEQ ID NO:2.

SEQ ID NO:2: VL region of the 629.3 mAb antibody FR1-CDR1-FR2-CDR2-FR3-CDR3-FR4

According to particular aspects of the disclosures, the antibodies of the present disclosure thus compete for binding to BTLA with the 629.3 mAb characterized in that the 629.3 mAb comprises a heavy chain wherein the VH region is identical to SEQ ID NO:1 and a light chain wherein the VL region is identical to SEQ ID NO:2.

The inventors further identified the conformational epitope recognized by the antibody of the disclosure (629.3 mAb) by means of full discontinuous epitope mapping. Said conformational epitope comprise the regions of SEQ ID NO:5 and SEQ ID NO:6.

SED ID NO:5: VKLEDRQTSWKEEKN

SED ID NO:6: PSKDEMASRPWLL

Accordingly, the present disclosure provides for an isolated anti-BTLA antibody, wherein said antibody binds to an epitope of the BTLA protein comprising residues VKLEDRQTSWKEEKN (SEQ ID NO:5) and PSKDEMASRPWLL (SEQ ID NO:6).

In one aspects of the disclosure, the present disclosure provides for an isolated anti-BTLA antibody, wherein said antibody binds to the same epitope as 629.3 mAb.

In one aspects of the disclosure, the present disclosure provides for an isolated anti-BTLA antibody, wherein said antibody binds to an epitope of the BTLA protein comprising residues VKLEDRQTSWKEEKN (SEQ ID NO:5) and PSKDEMASRPWLL (SEQ ID NO:6) and competes for binding to BTLA with the 629.3 mAb.

The term "epitope" refers to any protein determinant capable of specific binding to an immunoglobin or T-cell receptor.

In particular, the present disclosure thus provides antibodies comprising functional variants of the VL region, VH region, or one or more CDRs of the 629.3 mAb. A functional variant of a VL, VH, or CDR used in the context of a monoclonal antibody of the present disclosure still allows the antibody to retain at least a substantial proportion (at least about 50%, 60%, 70%, 80%, 90%, 95% or more) of the affinity/avidity and/or the specificity/selectivity of the parent antibody (i.e. 629.3 mAb antibody) and in some cases such a monoclonal antibody of the present disclosure may be associated with greater affinity, selectivity and/or specificity than the parent Ab. Such functional variants typically retain significant sequence identity to the parent Ab. The sequence of CDR variants may differ from the sequence of the CDR of the parent antibody sequences through mostly conservative substitutions; for instance at least about 35%, about 50% or more, about 60% or more, about 70% or more, about 75% or more, about 80% or more, about 85% or more, about 90% or more, (e.g., about 65-95%, such as about 92%, 93% or 94%) of the substitutions in the variant are conservative amino acid residue replacements. The sequences of CDR variants may differ from the sequence of the CDRs of the parent antibody sequences through mostly conservative substitutions; for instance at least 10, such as at least 9, 8, 7, 6, 5, 4, 3, 2 or 1 of the substitutions in the variant are conservative amino acid residue replacements. In the context of the present disclosure, conservative substitutions may be defined by substitutions within the classes of amino acids reflected as follows:
Aliphatic residues I, L, V, and M
Cycloalkenyl-associated residues F, H, W, and Y
Hydrophobic residues A, C, F, G, H, I, L, M, R, T, V, W, and Y
Negatively charged residues D and E
Polar residues C, D, E, H, K, N, Q, R, S, and T
Positively charged residues H, K, and R
Small residues A, C, D, G, N, P, S, T, and V
Very small residues A, G, and S
Residues involved in turn A, C, D, E, G, H, K, N, Q, R, S, P, and formation T
Flexible residues Q, T, K, S, G, P, D, E, and R

More conservative substitutions groupings include: valine-leucine-isoleucine, phenylalanine-tyrosine, lysine-arginine, alanine-valine, and asparagine-glutamine. Conservation in terms of hydropathic/hydrophilic properties and residue weight/size also is substantially retained in a variant CDR as compared to a CDR of the 629.3 mAb. The importance of the hydropathic amino acid index in conferring interactive biologic function on a protein is generally understood in the art. It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathic index on the basis of their hydrophobicity and charge characteristics these are: isoleucine (+4.5); valine (+4.2); leucine (+3.8) ; phenylalanine (+2.8); cysteine/cystine (+2.5); methionine (+1.9); alanine (+1.8); glycine (-0.4); threonine (-0.7); serine (-0.8); tryptophane (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5). The retention of similar residues may also or alternatively be measured by a similarity score, as determined by use of a BLAST program (e.g., BLAST 2.2.8 available through the NCBI using standard settings BLOSUM62, Open Gap= 11 and Extended Gap= 1). Suitable variants typically exhibit at least about 70% of identity to the parent peptide. According to the present disclosure a first amino acid sequence having at least 70% of identity with a second amino acid sequence means that the first sequence has 70; 71; 72; 73; 74; 75; 76; 77; 78; 79; 80; 81; 82; 83; 84; 85; 86; 87; 88; 89; 90; 91; 92; 93; 94; 95; 96; 97; 98; 99; or 100% of identity with the second amino acid sequence. According to the present disclosure a first amino acid sequence having at least 50% of identity with a second amino acid sequence means that the first sequence has 50; 51; 52; 53; 54; 55; 56; 57; 58; 59; 60; 61; 62; 63; 64; 65; 66; 67; 68; 69; 70; 71; 72; 73; 74; 75; 76; 77; 78; 79; 80; 81; 82; 83; 84; 85; 86; 87; 88; 89; 90; 91; 92; 93; 94; 95; 96; 97; 98; 99; or 100% of identity with the second amino acid sequence.

In some aspects of the disclosures, the antibody of the present disclosure is an antibody having a heavy chain comprising i) a H-CDR1 having at least 50% of identity with the H-CDR1 of the 629.3 mAb, ii) a H-CDR2 having at least 50% of identity with the H-CDR2 of the 629.3 mAb and iii) a H-CDR3 having at least 50% of identity with the H-CDR3 of the 629.3 mAb.

In some aspects of the disclosures, the antibody of the present disclosure is an antibody having a light chain comprising i) a L-CDR1 having at least 50% of identity with the L-CDR1 of the 629.3 mAb, ii) a L-CDR2 having at least 50% of identity with the L-CDR2 of the 629.3 mAb and iii) a L-CDR3 having at least 50% of identity with the L-CDR3 of the 629.3 mAb.

In some aspects of the disclosures, the antibody of the present disclosure is an antibody having a heavy chain comprising i) a H-CDR1 having at least 50% of identity with the H-CDR1 of the 629.3 mAb, ii) a H-CDR2 having at least 50% of identity with the H-CDR2 of the 629.3 mAb and iii) a H-CDR3 having at least 50% of identity with the H-CDR3 of the 629.3 mAb and a light chain comprising i) a L-CDR1 having at least 50% of identity with the L-CDR1 of the 629.3 mAb, ii) a L-CDR2 having at least 50% of identity with the L-CDR2 of the 629.3 mAb and iii) a L-CDR3 having at least 50% of identity with the L-CDR3 of the 629.3 mAb.

In some aspects of the disclosures, the antibody of the present disclosure is an antibody having a heavy chain comprising i) the H-CDR1 of the 629.3 mAb, ii) the H-CDR2 of the 629.3 mAb and iii) the H-CDR3 of the 629.3 mAb.

In some aspects of the disclosures, the antibody of the present disclosure is an antibody having a light chain comprising i) the L-CDR1 of the 629.3 mAb, ii) the L-CDR2 of the 629.3 mAb and iii) the L-CDR3 of the 629.3 mAb.

In some aspects of the disclosures, the antibody of the present disclosure is an antibody having a heavy chain comprising i) the H-CDR1 of the 629.3 mAb, ii) the H-CDR2 of the 629.3 mAb and iii) the H-CDR3 of the 629.3 mAb and a light chain comprising i) the L-CDR1 of the 629.3 mAb, ii) the L-CDR2 of the 629.3 mAb and iii) the L-CDR3 of the 629.3 mAb.

In some aspects of the disclosures, the antibody of the present disclosure is an antibody having a heavy chain wherein the VH region has at least 70% of identity with SEQ ID NO:1

In some aspects of the disclosures, the antibody of the present disclosure is an antibody having a light chain having at least 70% of identity with SEQ ID NO:2.

In some aspects of the disclosures, the antibody of the present disclosure is an antibody having a heavy chain wherein the VH region has at least 70% of identity with SEQ ID NO:1 and a light chain wherein the VL region has at least 70% of identity with SEQ ID NO:2.

In some aspects of the disclosures, the antibody of the present disclosure is an antibody having a heavy chain wherein the VH region is identical to SEQ ID NO: 1

In some aspects of the disclosures, the antibody of the present disclosure is an antibody having a light chain wherein the VL region is identical to SEQ ID NO:2.

In some aspects of the disclosures, the antibody of the present disclosure is an antibody having a heavy chain wherein the VH region is identical to SEQ ID NO: 1 and a light chain wherein the VL region is identical to SEQ ID NO:2.

In some aspects of the disclosures, the antibody of the present disclosure is a chimeric antibody, typically a chimeric mouse/human antibody. The term "chimeric antibody" refers to a monoclonal antibody which comprises a VH domain and a VL domain of an antibody derived from a non-human animal, a CH domain and a CL domain of a human antibody. As the non-human animal, any animal such as mouse, rat, hamster, rabbit or the like can be used. In particular, said mouse/human chimeric antibody may comprise the heavy chain and the light chain of the 629.3 mAb antibody.

In some aspects of the disclosures, the antibody of the present disclosure is a humanized antibody. In specific aspects of the disclosures, the antibody of the present disclosure is a humanized antibody which comprises the CDRs of the 629.3 mAb antibody. As used herein the term "humanized antibody" refers to antibodies in which the framework or "complementarity determining regions" (CDR) have been modified to comprise the CDR from a donor immunoglobulin of different specificity as compared to that of the parent immunoglobulin.

In some aspects of the disclosures, the antibody of the present disclosure is selected from the group of Fab, F(ab')2, Fab' and scFv. As used herein, the term "Fab" denotes an antibody fragment having a molecular weight of about 50,000 and antigen binding activity, in which about a half of the N-terminal side of H chain and the entire L chain, among fragments obtained by treating IgG with a protease, papaine, are bound together through a disulfide bond. The term "F(ab')2" refers to an antibody fragment having a molecular weight of about 100,000 and antigen binding activity, which is slightly larger than the Fab bound via a disulfide bond of the hinge region, among fragments obtained by treating IgG with a protease, pepsin. The term "Fab' " refers to an antibody fragment having a molecular weight of about 50,000 and antigen binding activity, which is obtained by cutting a disulfide bond of the hinge region of the F(ab')2. A single chain Fv ("scFv") polypeptide is a covalently linked VH::VL heterodimer which is usually expressed from a gene fusion including VH and VL encoding genes linked by a peptide-encoding linker. The human scFv fragment of the disclosure includes CDRs that are held in appropriate conformation, preferably by using gene recombination techniques.

The antibodies of the present disclosure are produced by any technique known in the art, such as, without limitation, any chemical, biological, genetic or enzymatic technique, either alone or in combination. Typically, knowing the amino acid sequence of the desired sequence, one skilled in the art can readily produce said antibodies, by standard techniques for production of polypeptides. For instance, they can be synthesized using well-known solid phase method, preferably using a commercially available peptide synthesis apparatus (such as that made by Applied Biosystems, Foster City, California) and following the manufacturer's instructions. Alternatively, antibodies of the present disclosure can be synthesized by recombinant DNA techniques well-known in the art. For example, antibodies can be obtained as DNA expression products after incorporation of DNA sequences encoding the antibodies into expression vectors and introduction of such vectors into suitable eukaryotic or prokaryotic hosts that will express the desired antibodies, from which they can be later isolated using well-known techniques.

Accordingly, a further object of the disclosure relates to a nucleic acid molecule encoding an antibody according to the disclosure. More particularly the nucleic acid molecule encodes a heavy chain or a light chain of an antibody of the present disclosure. More particularly the nucleic acid molecule comprises a nucleic acid sequence having 70% of identity with SEQ ID NO:3 or SEQ ID NO:4.

SEQ ID NO:3 VH region: DNA sequence FR1-**CDR1**-FR2-**CDR2**-FR3-**CDR3**-FR4

SEQ ID NO:4 VL region: DNA sequence FR1-**CDR1**-FR2-**CDR2**-FR3-**CDR3**-FR4

Typically, said nucleic acid is a DNA or RNA molecule, which may be included in any suitable vector, such as a plasmid, cosmid, episome, artificial chromosome, phage or a viral vector. As used herein, the terms "vector", "cloning vector" and "expression vector" mean the vehicle by which a DNA or RNA sequence (e.g. a foreign gene) can be introduced into a host cell, so as to transform the host and promote expression (e.g. transcription and translation) of the introduced sequence. So, a further object of the disclosure relates to a vector comprising a nucleic acid of the disclosure. Such vectors may comprise regulatory elements, such as a promoter, enhancer, terminator and the like, to cause or direct expression of said antibody upon administration to a subject. Examples of promoters and enhancers used in the expression vector for animal cell include early promoter and enhancer of SV40, LTR promoter and enhancer of Moloney mouse leukemia virus, promoter and enhancer of immunoglobulin H chain and the like. Any expression vector for animal cell can be used, so long as a gene encoding the human antibody C region can be inserted and expressed. Examples of suitable vectors include pAGE107, pAGE103, pHSG274, pKCR, pSG1 beta d2-4 and the like. Other examples of plasmids include replicating plasmids comprising an origin of replication, or integrative plasmids, such as for instance pUC, pcDNA, pBR, and the like. Other examples of viral vector include adenoviral, retroviral, herpes virus and AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO 95/14785, WO 96/22378, US 5,882,877, US 6,013,516, US 4,861,719, US 5,278,056 and WO 94/19478.

A further object of the present disclosure relates to a host cell which has been transfected, infected or transformed by a nucleic acid and/or a vector according to the disclosure. As used herein, the term "transformation" means the introduction of a "foreign" (i.e. extrinsic or extracellular) gene, DNA or RNA sequence to a host cell, so that the host cell will express the introduced gene or sequence to produce a desired substance, typically a protein or enzyme coded by the introduced gene or sequence. A host cell that receives and expresses introduced DNA or RNA bas been "transformed".

The nucleic acids of the disclosure may be used to produce an antibody of the present disclosure in a suitable expression system. The term "expression system" means a host cell and compatible vector under suitable conditions, e.g. for the expression of a protein coded for by foreign DNA carried by the vector and introduced to the host cell. Common expression systems include E. coli host cells and plasmid vectors, insect host cells and Baculovirus vectors, and mammalian host cells and vectors. Other examples of host cells include, without limitation, prokaryotic cells (such as bacteria) and eukaryotic cells (such as yeast cells, mammalian cells, insect cells, plant cells, etc.). Specific examples include E.coli, Kluyveromyces or Saccharomyces yeasts, mammalian cell lines (e.g., Vero cells, CHO cells, 3T3 cells, COS cells, etc.) as well as primary or established mammalian cell cultures (e.g., produced from lymphoblasts, fibroblasts, embryonic cells, epithelial cells, nervous cells, adipocytes, etc.). Examples also include mouse SP2/0-Ag14 cell (ATCC CRL1581), mouse P3X63-Ag8.653 cell (ATCC CRL1580), CHO cell in which a dihydrofolate reductase gene (hereinafter referred to as "DHFR gene") is defective (Urlaub G et al; 1980), rat YB2/3HL.P2.G11.16Ag.20 cell (ATCC CRL1662, hereinafter referred to as "YB2/0 cell"), and the like. The present disclosure also relates to a method of producing a recombinant host cell expressing an antibody according to the disclosure, said method comprising the steps of: (i) introducing in vitro or ex vivo a recombinant nucleic acid or a vector as described above into a competent host cell, (ii) culturing in vitro or ex vivo the recombinant host cell obtained and (iii), optionally, selecting the cells which express and/or secrete said antibody. Such recombinant host cells can be used for the production of antibodies of the present disclosure.

Antibodies of the present disclosure are suitably separated from the culture medium by conventional immunoglobulin purification procedures such as, for example, protein A-Sepharose, hydroxylapatite chromatography, gel electrophoresis, dialysis, or affinity chromatography.

In some aspects of the disclosures, the human chimeric antibody of the present disclosure can be produced by obtaining nucleic sequences encoding VL and VH domains as previously described, constructing a human chimeric antibody expression vector by inserting them into an expression vector for animal cell having genes encoding human antibody CH and human antibody CL, and expressing the coding sequence by introducing the expression vector into an animal cell. As the CH domain of a human chimeric antibody, it may be any region which belongs to human immunoglobulin, but those of IgG class are suitable and any one of subclasses belonging to IgG class, such as IgG1, IgG2, IgG3 and IgG4, can also be used. Also, as the CL of a human chimeric antibody, it may be any region which belongs to Ig, and those of kappa class or lambda class can be used. Methods for producing chimeric antibodies involve conventional recombinant DNA and gene transfection techniques are well known in the art (See Morrison SL. et al. (1984) and patent documents US5,202,238; and US5,204, 244).

The humanized antibody of the present disclosure may be produced by obtaining nucleic acid sequences encoding CDR domains, as previously described, constructing a humanized antibody expression vector by inserting them into an expression vector for animal cell having genes encoding (i) a heavy chain constant region identical to that of a human antibody and (ii) a light chain constant region identical to that of a human antibody, and expressing the genes by introducing the expression vector into an animal cell. The humanized antibody expression vector may be either of a type in which a gene encoding an antibody heavy chain and a gene encoding an antibody light chain exists on separate vectors or of a type in which both genes exist on the same vector (tandem type). In respect of easiness of construction of a humanized antibody expression vector, easiness of introduction into animal cells, and balance between the expression levels of antibody H and L chains in animal cells, humanized antibody expression vector of the tandem type is preferred. Examples of tandem type humanized antibody expression vector include pKANTEX93 (WO 97/10354), pEE18 and the like. Methods for producing humanized antibodies based on conventional recombinant DNA and gene transfection techniques are well known in the art (See, e. g., Riechmann L. et al. 1988; Neuberger MS. et al. 1985). Antibodies can be humanized using a variety of techniques known in the art including, for example, CDR-grafting (EP 239,400; PCT publication WO91/09967; U.S. Pat. Nos. 5,225,539; 5,530,101; and 5,585,089), veneering or resurfacing (EP 592,106; EP 519,596; Padlan EA (1991); Studnicka GM et al. (1994); Roguska MA. et al. (1994)), and chain shuffling (U.S. Pat. No.5,565,332). The general recombinant DNA technology for preparation of such antibodies is also known (see European Patent Application EP 125023 and International Patent Application WO 96/02576).

The Fab of the present disclosure can be obtained by treating an antibody which specifically reacts with AMH with a protease, papaine. Also, the Fab can be produced by inserting DNA encoding Fab of the antibody into a vector for prokaryotic expression system, or for eukaryotic expression system, and introducing the vector into a procaryote or eucaryote (as appropriate) to express the Fab.

The F(ab')2 of the present disclosure can be obtained treating an antibody which specifically reacts with AMH with a protease, pepsin. Also, the F(ab')2 can be produced by binding Fab' described below via a thioether bond or a disulfide bond.

The Fab' of the present disclosure can be obtained treating F(ab')2 which specifically reacts with AMH with a reducing agent, dithiothreitol. Also, the Fab' can be produced by inserting DNA encoding Fab' fragment of the antibody into an expression vector for prokaryote, or an expression vector for eukaryote, and introducing the vector into a prokaryote or eukaryote (as appropriate) to perform its expression.

The scFv of the present disclosure can be produced by obtaining cDNA encoding the VH and VL domains as previously described, constructing DNA encoding scFv, inserting the DNA into an expression vector for prokaryote, or an expression vector for eukaryote, and then introducing the expression vector into a prokaryote or eukaryote (as appropriate) to express the scFv. To generate a humanized scFv fragment, a well known technology called CDR grafting may be used, which involves selecting the complementary determining regions (CDRs) from a donor scFv fragment, and grafting them onto a human scFv fragment framework of known three dimensional structure (see, e. g., WO98/45322; WO 87/02671; US5,859,205; US5,585,089; US4,816,567; EP0173494).

Engineered antibodies of the present disclosure include those in which modifications have been made to framework residues within VH and/or VL, e.g. to improve the properties of the antibody. Typically such framework modifications are made to decrease the immunogenicity of the antibody. For example, one approach is to "backmutate" one or more framework residues to the corresponding germline sequence. More specifically, an antibody that has undergone somatic mutation may contain framework residues that differ from the germline sequence from which the antibody is derived. Such residues can be identified by comparing the antibody framework sequences to the germline sequences from which the antibody is derived. To return the framework region sequences to their germline configuration, the somatic mutations can be "backmutated" to the germline sequence by, for example, site-directed mutagenesis or PCR-mediated mutagenesis. Such "backmutated" antibodies are also intended to be encompassed by the disclosure. Another type of framework modification involves mutating one or more residues within the framework region, or even within one or more CDR regions, to remove T cell -epitopes to thereby reduce the potential immunogenicity of the antibody. This approach is also referred to as "deimmunization" and is described in further detail in U.S. Patent Publication No. 20030153043 by Carr et al.

The antibody of the disclosure can be characterized by one or more of the functional or structural features of the aspects described above, or by any combination of selected functional and structural features.

The antibody of the disclosure may be of any isotype. The choice of isotype typically will be guided by the desired effector functions, such as ADCC induction. Exemplary isotypes are IgG1, IgG2, IgG3, and IgG4. Either of the human light chain constant regions, kappa or lambda, may be used. If desired, the class of an antibody of the present disclosure may be switched by known methods. Typical, class switching techniques may be used to convert one IgG subclass to another, for instance from IgG1 to IgG2. Thus, the effector function of the antibodies of the present disclosure may be changed by isotype switching to, e.g., an IgG1, IgG2, IgG3, IgG4, IgD, IgA, IgE, or IgM antibody for various therapeutic uses. In some aspects of the disclosures, the antibody of the disclosure is a full-length antibody. In some aspects of the disclosures, the full-length antibody is an IgG1 antibody. In some aspects of the disclosures, the full-length antibody is an IgG4 antibody. In some aspects of the disclosures, the BTLA-specific IgG4 antibody is a stabilized IgG4 antibody. Examples of suitable stabilized IgG4 antibodies are antibodies wherein arginine at position 409 in a heavy chain constant region of human IgG4, which is indicated in the EU index as in Kabat et al. supra, is substituted with lysine, threonine, methionine, or leucine, preferably lysine (described in WO2006033386) and/or wherein the hinge region comprises a Cys-Pro-Pro-Cys sequence. Other suitable stabilized IgG4 antbodies are disclosed in WO2008145142, which is hereby incorporated by reference in its entirety.

In some aspects of the disclosures, the antibody of the present disclosure does not comprise a Fc portion that induces antibody dependent cellular cytotoxicity (ADCC). The terms "Fc domain," "Fc portion," and "Fc region" refer to a C-terminal fragment of an antibody heavy chain, e.g., from about amino acid (aa) 230 to about aa 450 of human gamma heavy chain or its counterpart sequence in other types of antibody heavy chains (e.g., α, δ, ε and µ for human antibodies), or a naturally occurring allotype thereof. Unless otherwise specified, the commonly accepted Kabat amino acid numbering for immunoglobulins is used throughout this disclosure (see Kabat et al. (1991) Sequences of Protein of Immunological Interest, 5th ed., United States Public Health Service, National Institute of Health, Bethesda, MD). In some aspects of the disclosures, the antibody of the present disclosure does not comprise an Fc domain capable of substantially binding to a FcgRIIIA (CD16) polypeptide. In some aspects of the disclosures, the antibody of the present disclosure lacks an Fc domain (e.g. lacks a CH2 and/or CH3 domain) or comprises an Fc domain of IgG2 or IgG4 isotype. In some aspects of the disclosures, the antibody of the present disclosure consists of or comprises a Fab, Fab', Fab'-SH, F (ab') 2, Fv, a diabody, single-chain antibody fragment, or a multispecific antibody comprising multiple different antibody fragments. In some aspects of the disclosures, the antibody of the present disclosure is not linked to a toxic moiety. In some aspects of the disclosures, one or more amino acids selected from amino acid residues can be replaced with a different amino acid residue such that the antibody has altered C2q binding and/or reduced or abolished complement dependent cytotoxicity (CDC). This approach is described in further detail in U.S. Patent Nos. 6,194,551.

Another modification of the antibodies herein that is contemplated by the disclosure is pegylation. An antibody can be pegylated to, for example, increase the biological (e.g., serum) half-life of the antibody. To pegylate an antibody, the antibody, or fragment thereof, typically is reacted with polyethylene glycol (PEG), such as a reactive ester or aldehyde derivative of PEG, under conditions in which one or more PEG groups become attached to the antibody or antibody fragment. The pegylation can be carried out by an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (CI- CIO) alkoxy- or aryloxy-poly ethylene glycol or polyethylene glycol-maleimide. In some aspects of the disclosures, the antibody to be pegylated is an aglycosylated antibody. Methods for pegylating proteins are known in the art and can be applied to the antibodies of the present disclosure. See for example, EP 0154 316 by Nishimura et al. and EP 0 401 384 by Ishikawa et al.

Another modification of the antibodies that is contemplated by the disclosure is a conjugate or a protein fusion of at least the antigen-binding region of the antibody of the present disclosure to serum protein, such as human serum albumin or a fragment thereof to increase half-life of the resulting molecule.

In some aspects of the disclosures, the disclosure provides a multispecific antibody. Exemplary formats for the multispecific antibody molecules of the disclosure include, but are not limited to (i) two antibodies cross-linked by chemical heteroconjugation, one with a specificity to BTLA and another with a specificity to a second antigen; (ii) a single antibody that comprises two different antigen-binding regions; (iii) a single-chain antibody that comprises two different antigen-binding regions, e.g., two scFvs linked in tandem by an extra peptide linker; (iv) a dual-variable-domain antibody (DVD-Ig), where each light chain and heavy chain contains two variable domains in tandem through a short peptide linkage (Wu et al., Generation and Characterization of a Dual Variable Domain Immunoglobulin (DVD-Ig™) Molecule, In : Antibody Engineering, Springer Berlin Heidelberg (2010)); (v) a chemically-linked bispecific (Fab')2 fragment; (vi) a Tandab, which is a fusion of two single chain diabodies resulting in a tetravalent bispecific antibody that has two binding sites for each of the target antigens; (vii) a flexibody, which is a combination of scFvs with a diabody resulting in a multivalent molecule; (viii) a so called "dock and lock" molecule, based on the "dimerization and docking domain" in Protein Kinase A, which, when applied to Fabs, can yield a trivaient bispecific binding protein consisting of two identical Fab fragments linked to a different Fab fragment; (ix) a so-called Scorpion molecule, comprising, e.g., two scFvs fused to both termini of a human Fab-arm; and (x) a diabody. Another exemplary format for bispecific antibodies is IgG-like molecules with complementary CH3 domains to force heterodimerization. Such molecules can be prepared using known technologies, such as, e.g., those known as Triomab/Quadroma (Trion Pharma/Fresenius Biotech), Knob-into-Hole (Genentech), CrossMAb (Roche) and electrostatically-matched (Amgen), LUZ-Y (Genentech), Strand Exchange Engineered Domain body (SEEDbody)(EMD Serono), Biclonic (Merus) and DuoBody (Genmab A/S) technologies. In some aspects of the disclosures, the bispecific antibody is obtained or obtainable via a controlled Fab-arm exchange, typically using DuoBody technology. In vitro methods for producing bispecific antibodies by controlled Fab-arm exchange have been described in WO2008119353 and WO 2011131746 (both by Genmab A/S). In one exemplary method, described in WO 2008119353, a bispecific antibody is formed by "Fab-arm" or "half- molecule" exchange (swapping of a heavy chain and attached light chain) between two monospecific antibodies, both comprising IgG4-like CH3 regions, upon incubation under reducing conditions. The resulting product is a bispecific antibody having two Fab arms which may comprise different sequences. In another exemplary method, described in WO 2011131746, bispecific antibodies of the present disclosure are prepared by a method comprising the following steps, wherein at least one of the first and second antibodies is the antibody of the present disclosure : a) providing a first antibody comprising an Fc region of an immunoglobulin, said Fc region comprising a first CH3 region; b) providing a second antibody comprising an Fc region of an immunoglobulin, said Fc region comprising a second CH3 region; wherein the sequences of said first and second CH3 regions are different and are such that the heterodimeric interaction between said first and second CH3 regions is stronger than each of the homodimeric interactions of said first and second CH3 regions; c) incubating said first antibody together with said second antibody under reducing conditions; and d) obtaining said bispecific antibody, wherein the first antibody is the antibody of the present disclosure and the second antibody has a different binding specificity, or vice versa. The reducing conditions may, for example, be provided by adding a reducing agent, e.g. selected from 2-mercaptoethylamine, dithiothreitol and tris(2-carboxyethyl)phosphine. Step d) may further comprise restoring the conditions to become non-reducing or less reducing, for example by removal of a reducing agent, e.g. by desalting. Preferably, the sequences of the first and second CH3 regions are different, comprising only a few, fairly conservative, asymmetrical mutations, such that the heterodimeric interaction between said first and second CH3 regions is stronger than each of the homodimeric interactions of said first and second CH3 regions. More details on these interactions and how they can be achieved are provided in WO 2011131746, which is hereby incorporated by reference in its entirety. The following are exemplary aspects of the disclosures of combinations of such assymetrical mutations, optionally wherein one or both Fc-regions are of the IgG1 isotype.

Accordingly, one object of the present disclosure relates to a method of increasing the proliferation of Vγ9Vδ2 T cells in a subject in need thereof comprising administering to the subject a therapeutically effective amount of an antibody of the present disclosure.

As used herein the term "Vγ9Vδ2 T cell" has its general meaning in the art. Vγ9Vδ2 cells, the major γδ T cell subset in human peripheral blood, represent a T-cell subset displaying reactivity against microbial agents and tumors.

In some aspects of the disclosures, the subject suffers from a cancer or an infectious disease. Accordingly, a further object of the present disclosure relates to a method of treating a cancer or an infectious disease in a subject in need thereof comprising administering to the subject a therapeutically effective amount of an antibody of the present disclosure.

As used herein, the term "treatment" or "treat" refer to both prophylactic or preventive treatment as well as curative or disease modifying treatment, including treatment of subject at risk of contracting the disease or suspected to have contracted the disease as well as subjects who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a subject during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a subject during treatment of an illness, e.g., to keep the subject in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., disease manifestation, etc.]).

As used herein, the term "cancer" has its general meaning in the art and includes, but is not limited to, solid tumors and blood borne tumors The term cancer includes diseases of the skin, tissues, organs, bone, cartilage, blood and vessels. The term "cancer" further encompasses both primary and metastatic cancers. Examples of cancers that may treated by methods and compositions of the disclosure include, but are not limited to, cancer cells from the bladder, blood, bone, bone marrow, brain, breast, colon, esophagus, gastrointestinal, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous; adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; the coma, malignant; granulosa cell tumor, malignant; and roblastoma, malignant; Sertoli cell carcinoma; leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malign melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia.

In some aspects of the disclosures, the antibody of the present disclosure is used in a method of treatment of a haematological malignancy. Hematologic malignancies include but are not limited to leukemias including acute myeloid leukemias, acute lymphoid leukemias, multiple myeloma, lymphoid cell neoplasms such as chronic lymphocytic leukaemia (CLL), non-Hodgkin lymphoma (NHL), small lymphocytic lymphoma (SLL), and mantle cell lymphoma (MCL). More specifically, non-Hodgkin lymphoma (NHL) include B and T non-Hodgkin lymphoma. Furthermore, cell lymphoid neoplasms include B, NK and T cell lymphoid neoplasms. Preferably, said haematological malignancy is a lymphoma. Indeed, the inventors have put in light that BTLA stimulation of Vγ9Vδ2T cell is a highly promising mechanism of immune escape by lymphoma cells. More preferably, said lymphoma is selected among Non-Hodgkin lymphomas and Hodgkin lymphomas. As used herein, the term "leukemia" has generally been used to define hematologic malignancies of the blood or bone marrow characterized by abnormal proliferation of leukocytes. The principal subtypes of leukemia are identified on the basis of malignancy involving lymphoid (e.g. T or B lymphocytic lineage) or myeloid (e.g. granulocytic, erythroid or megakaryocytic lineage) cells, and whether the disease is acute or chronic in onset [Freireich, E.J. et al., 1991]. As used herein, the term "lymphoma" covers a heterogeneous group of neoplasms of lymphoid tissue. Lymphomas are broadly categorized under Hodgkin lymphoma, and T-cell (T-NHL) and B-cell (B-NHL) non-Hodgkin lymphomas. A World Health Organization (WHO) classification has recently been published (discussed later in this application), and diagnostic guidelines have been established based on this classification [Jaffe, E.S. et al., 2004]. Chronic Lymphocytic Leukemia (CLL) is a form of lymphocytic leukemia characterized by slow but progressive accumulation of lymphocytes in the bone marrow and blood. Depending on the stage of the disease, lymph node and spleen enlargement occur commonly. Although CLL may be of T cell or B cell origin, over 85% of the cases are of B-cell origin. Current understanding suggests that CLL is a heterogeneous disease originating from B lymphocytes that differ in their activation and maturation states and cellular subgroup (see [Kuppers, R., 2005]). The disease may result both from decreased apoptosis as well as increased proliferation of the leukemic B cells. CLL cells are usually clonal in origin, and express the following cell surface markers: CD19, CD20, CD21, and CD24. In addition, they express CD5 which is more typically found on T cells (see [Chiorazzi, N, and al., 2005]). CLL is considered a subgroup of "non-Hodgkin's lymphoma" (NHL) and together with the closely related disease "small lymphocytic lymphoma" (SLL) which presents primarily in the lymph nodes, corresponds to around 20% of all NHL cases. CLL is the most common leukemia in adults in the US and most of Western Europe. The National Cancer Institute (NCI) estimate for the incidence of CLL is about 10.000 new cases in the US per year. Clinical manifestations of CLL occur predominantly after the age of 55. The incidence rate for men is higher than for women, with men almost twice as likely to acquire the disease as women. CLL represents an unmet medical need as there are limited options for treatment.

In some aspects of the disclosures, the antibody of the present disclosure is used in a method of treatment of an infectious disease. As used herein the term "infectious disease" includes any infection caused by viruses, bacteria, protozoa, molds or fungi. In some aspects of the disclosures, the viral infection comprises infection by one or more viruses selected from the group consisting of Arenaviridae, Astroviridae, Birnaviridae, Bromoviridae, Bunyaviridae, Caliciviridae, Closteroviridae, Comoviridae, Cystoviridae, Flaviviridae, Flexiviridae, Hepevirus, Leviviridae, Luteoviridae, Mononegavirales, Mosaic Viruses, Nidovirales, Nodaviridae, Orthomyxoviridae, Picobirnavirus, Picornaviridae, Potyviridae, Reoviridae, Retroviridae, Sequiviridae, Tenuivirus, Togaviridae, Tombusviridae, Totiviridae, Tymoviridae, Hepadnaviridae, Herpesviridae, Paramyxoviridae or Papillomaviridae viruses. Relevant taxonomic families of RNA viruses include, without limitation, Astroviridae, Birnaviridae, Bromoviridae, Caliciviridae, Closteroviridae, Comoviridae, Cystoviridae, Flaviviridae, Flexiviridae, Hepevirus, Leviviridae, Luteoviridae, Mononegavirales, Mosaic Viruses, Nidovirales, Nodaviridae, Orthomyxoviridae, Picobirnavirus, Picornaviridae, Potyviridae, Reoviridae, Retroviridae, Sequiviridae, Tenuivirus, Togaviridae, Tombusviridae, Totiviridae, and Tymoviridae viruses. In some aspects of the disclosures, the viral infection comprises infection by one or more viruses selected from the group consisting of adenovirus, rhinovirus, hepatitis, immunodeficiency virus, polio, measles, Ebola, Coxsackie, Rhino, West Nile, small pox, encephalitis, yellow fever, Dengue fever, influenza (including human, avian, and swine), lassa, lymphocytic choriomeningitis, junin, machuppo, guanarito, hantavirus, Rift Valley Fever, La Crosse, California encephalitis, Crimean-Congo, Marburg, Japanese Encephalitis, Kyasanur Forest, Venezuelan equine encephalitis, Eastern equine encephalitis, Western equine encephalitis, severe acute respiratory syndrome (SARS), parainfluenza, respiratory syncytial, Punta Toro, Tacaribe, pachindae viruses, adenovirus, Dengue fever, influenza A and influenza B (including human, avian, and swine), junin, measles, parainfluenza, Pichinde, punta toro, respiratory syncytial, rhinovirus, Rift Valley Fever, severe acute respiratory syndrome (SARS), Tacaribe, Venezuelan equine encephalitis, West Nile and yellow fever viruses, tick-borne encephalitis virus, Japanese encephalitis virus, St. Louis encephalitis virus, Murray Valley virus, Powassan virus, Rocio virus, louping-ill virus, Banzi virus, Ilheus virus, Kokobera virus, Kunjin virus, Alfuy virus, bovine diarrhea virus, and Kyasanur forest disease. Bacterial infections that can be treated according to this disclosure include, but are not limited to, infections caused by the following: Staphylococcus; Streptococcus, including S. pyogenes; Enterococcl; Bacillus, including Bacillus anthracis, and Lactobacillus; Listeria; Corynebacterium diphtheriae; Gardnerella including G. vaginalis; Nocardia; Streptomyces; Thermoactinomyces vulgaris; Treponerna; Camplyobacter, Pseudomonas including aeruginosa; Legionella; Neisseria including N.gonorrhoeae and N.meningitides; Flavobacterium including F. meningosepticum and F. odoraturn; Brucella; Bordetella including B. pertussis and B. bronchiseptica; Escherichia including E. coli, Klebsiella; Enterobacter, Serratia including S. marcescens and S. liquefaciens; Edwardsiella; Proteus including P. mirabilis and P. vulgaris; Streptobacillus; Rickettsiaceae including R. fickettsfi, Chlamydia including C. psittaci and C. trachomatis; Mycobacterium including M. tuberculosis, M. intracellulare, M. folluiturn, M. laprae, M. avium, M. bovis, M. africanum, M. kansasii, M. intracellulare, and M. lepraernurium; and Nocardia. Protozoa infections that may be treated according to this disclosure include, but are not limited to, infections caused by leishmania, kokzidioa, and trypanosoma. A complete list of infectious diseases can be found on the website of the National Center for Infectious Disease (NCID) at the Center for Disease Control (CDC) (World Wide Web (www) at cdc.gov/ncidod/diseases/), which list is incorporated herein by reference. All of said diseases are candidates for treatment using the compositions according to the disclosure.

In some aspects of the disclosures, the antibody of the present disclosure is used in combination with a chemotherapeutic agent. The term "chemotherapeutic agent" refers to chemical compounds that are effective in inhibiting tumor growth. Examples of chemotherapeutic agents include alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaorarnide and trimethylolomelamine; acetogenins (especially bullatacin and bullatacinone); a carnptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estrarnustine, ifosfamide, mechlorethamine, mechlorethamine oxide hydrochloride, melphalan, novembichin, phenesterine, prednimus tine, trofosfamide, uracil mustard; nitrosureas such as carmustine, chlorozotocin, fotemustine, lomustine, nimustine, ranimustine; antibiotics such as the enediyne antibiotics (e.g. calicheamicin, especially calicheamicin (11 and calicheamicin 211, see, e.g., Agnew Chem Intl. Ed. Engl. 33:183-186 (1994); dynemicin, including dynemicin A; an esperamicin; as well as neocarzinostatin chromophore and related chromoprotein enediyne antiobiotic chromomophores), aclacinomysins, actinomycin, authramycin, azaserine, bleomycins, cactinomycin, carabicin, canninomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idanrbicin, marcellomycin, mitomycins, mycophenolic acid, nogalarnycin, olivomycins, peplomycin, potfiromycin, puromycin, quelamycin, rodorubicin, streptomgrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophospharnide glycoside; aminolevulinic acid; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pento statin; phenamet; pirarubicin; podophyllinic acid; 2-ethylhydrazide; procarbazine; PSK®; razoxane; rhizoxin; sizofiran; spirogennanium; tenuazonic acid; triaziquone; 2,2',2"-trichlorotriethylarnine; trichothecenes (especially T-2 toxin, verracurin A, roridinA and anguidine); urethan; vindesine; dacarbazine; mannomustine; mitobromtol; mitolactol; pipobroman; gacytosine; arabinoside ("Ara-C"); cyclophosphamide; thiotepa; taxoids, e.g. paclitaxel (TAXOL®, Bristol-Myers Squibb Oncology, Princeton, N.].) and doxetaxel (TAXOTERE®, Rhone-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-1 1 ; topoisomerase inhibitor RFS 2000; difluoromethylornithine (DMFO); retinoic acid; capecitabine; and phannaceutically acceptable salts, acids or derivatives of any of the above. Also included in this definition are antihormonal agents that act to regulate or inhibit honnone action on tumors such as antiestrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and phannaceutically acceptable salts, acids or derivatives of any of the above.

In some aspects of the disclosures, the antibody of the present disclosure is used in combination with a targeted cancer therapy. Targeted cancer therapies are drugs or other substances that block the growth and spread of cancer by interfering with specific molecules ("molecular targets") that are involved in the growth, progression, and spread of cancer. Targeted cancer therapies are sometimes called "molecularly targeted drugs," "molecularly targeted therapies," "precision medicines," or similar names. In some aspects of the disclosures, the targeted therapy consists of administering the subject with a tyrosine kinase inhibitor. The term "tyrosine kinase inhibitor" refers to any of a variety of therapeutic agents or drugs that act as selective or non-selective inhibitors of receptor and/or non-receptor tyrosine kinases. Tyrosine kinase inhibitors and related compounds are well known in the art and described in U.S Patent Publication 2007/0254295, which is incorporated by reference herein in its entirety. It will be appreciated by one of skill in the art that a compound related to a tyrosine kinase inhibitor will recapitulate the effect of the tyrosine kinase inhibitor, e.g., the related compound will act on a different member of the tyrosine kinase signaling pathway to produce the same effect as would a tyrosine kinase inhibitor of that tyrosine kinase. Examples of tyrosine kinase inhibitors and related compounds suitable for use in methods of aspects of the disclosures of the present disclosure include, but are not limited to, dasatinib (BMS-354825), PP2, BEZ235, saracatinib, gefitinib (Iressa), sunitinib (Sutent; SU11248), erlotinib (Tarceva; OSI-1774), lapatinib (GW572016; GW2016), canertinib (CI 1033), semaxinib (SU5416), vatalanib (PTK787/ZK222584), sorafenib (BAY 43-9006), imatinib (Gleevec; STI571), leflunomide (SU101), vandetanib (Zactima; ZD6474), MK-2206 (8-[4-aminocyclobutyl)phenyl]-9-phenyl-1,2,4-triazolo[3,4-f][1,6]naphthyridin-3(2H)-one hydrochloride) derivatives thereof, analogs thereof, and combinations thereof. Additional tyrosine kinase inhibitors and related compounds suitable for use in the present disclosure are described in, for example, U.S Patent Publication 2007/0254295, U.S. Pat. Nos. 5,618,829, 5,639,757, 5,728,868, 5,804,396, 6,100,254, 6,127,374, 6,245,759, 6,306,874, 6,313,138, 6,316,444, 6,329,380, 6,344,459, 6,420,382, 6,479,512, 6,498,165, 6,544,988, 6,562,818, 6,586,423, 6,586,424, 6,740,665, 6,794,393, 6,875,767, 6,927,293, and 6,958,340, all of which are incorporated by reference herein in their entirety. In some aspects of the disclosures, the tyrosine kinase inhibitor is a small molecule kinase inhibitor that has been orally administered and that has been the subject of at least one Phase I clinical trial, more preferably at least one Phase II clinical, even more preferably at least one Phase III clinical trial, and most preferably approved by the FDA for at least one hematological or oncological indication. Examples of such inhibitors include, but are not limited to, Gefitinib, Erlotinib, Lapatinib, Canertinib, BMS-599626 (AC-480), Neratinib, KRN-633, CEP-11981, Imatinib, Nilotinib, Dasatinib, AZM-475271, CP-724714, TAK-165, Sunitinib, Vatalanib, CP-547632, Vandetanib, Bosutinib, Lestaurtinib, Tandutinib, Midostaurin, Enzastaurin, AEE-788, Pazopanib, Axitinib, Motasenib, OSI-930, Cediranib, KRN-951, Dovitinib, Seliciclib, SNS-032, PD-0332991, MKC-I (Ro-317453; R-440), Sorafenib, ABT-869, Brivanib (BMS-582664), SU-14813, Telatinib, SU-6668, (TSU-68), L-21649, MLN-8054, AEW-541, and PD-0325901.

In some aspects of the disclosures, the antibody of the present disclosure is used in combination with an immunotherapeutic agent. The term "immunotherapeutic agent," as used herein, refers to a compound, composition or treatment that indirectly or directly enhances, stimulates or increases the body's immune response against cancer cells and/or that decreases the side effects of other anticancer therapies. Immunotherapy is thus a therapy that directly or indirectly stimulates or enhances the immune system's responses to cancer cells and/or lessens the side effects that may have been caused by other anti-cancer agents. Immunotherapy is also referred to in the art as immunologic therapy, biological therapy biological response modifier therapy and biotherapy. Examples of common immunotherapeutic agents known in the art include, but are not limited to, cytokines, cancer vaccines, monoclonal antibodies and non-cytokine adjuvants. Alternatively the immunotherapeutic treatment may consist of administering the subject with an amount of immune cells (T cells, NK, cells, dendritic cells, B cells...). Immunotherapeutic agents can be non-specific, i.e. boost the immune system generally so that the human body becomes more effective in fighting the growth and/or spread of cancer cells, or they can be specific, i.e. targeted to the cancer cells themselves immunotherapy regimens may combine the use of non-specific and specific immunotherapeutic agents. Non-specific immunotherapeutic agents are substances that stimulate or indirectly improve the immune system. Non-specific immunotherapeutic agents have been used alone as a main therapy for the treatment of cancer, as well as in addition to a main therapy, in which case the non-specific immunotherapeutic agent functions as an adjuvant to enhance the effectiveness of other therapies (e.g. cancer vaccines). Non-specific immunotherapeutic agents can also function in this latter context to reduce the side effects of other therapies, for example, bone marrow suppression induced by certain chemotherapeutic agents. Non-specific immunotherapeutic agents can act on key immune system cells and cause secondary responses, such as increased production of cytokines and immunoglobulins. Alternatively, the agents can themselves comprise cytokines. Non-specific immunotherapeutic agents are generally classified as cytokines or non-cytokine adjuvants. A number of cytokines have found application in the treatment of cancer either as general non-specific immunotherapies designed to boost the immune system, or as adjuvants provided with other therapies. Suitable cytokines include, but are not limited to, interferons, interleukins and colony-stimulating factors. Interferons (IFNs) contemplated by the present disclosure include the common types of IFNs, IFN-alpha (IFN-α), IFN-beta (IFN-β) and IFN-gamma (IFN-γ). IFNs can act directly on cancer cells, for example, by slowing their growth, promoting their development into cells with more normal behavior and/or increasing their production of antigens thus making the cancer cells easier for the immune system to recognise and destroy. IFNs can also act indirectly on cancer cells, for example, by slowing down angiogenesis, boosting the immune system and/or stimulating natural killer (NK) cells, T cells and macrophages. Recombinant IFN-alpha is available commercially as Roferon (Roche Pharmaceuticals) and Intron A (Schering Corporation). Interleukins contemplated by the present disclosure include IL-2, IL-4, IL-11 and IL-12. Examples of commercially available recombinant interleukins include Proleukin® (IL-2; Chiron Corporation) and Neumega® (IL-12; Wyeth Pharmaceuticals). Zymogenetics, Inc. (Seattle, Wash.) is currently testing a recombinant form of IL-21, which is also contemplated for use in the combinations of the present disclosure. Colony-stimulating factors (CSFs) contemplated by the present disclosure include granulocyte colony stimulating factor (G-CSF or filgrastim), granulocyte-macrophage colony stimulating factor (GM-CSF or sargramostim) and erythropoietin (epoetin alfa, darbepoietin). Treatment with one or more growth factors can help to stimulate the generation of new blood cells in subjects undergoing traditional chemotherapy. Accordingly, treatment with CSFs can be helpful in decreasing the side effects associated with chemotherapy and can allow for higher doses of chemotherapeutic agents to be used. Various-recombinant colony stimulating factors are available commercially, for example, Neupogen® (G-CSF; Amgen), Neulasta (pelfilgrastim; Amgen), Leukine (GM-CSF; Berlex), Procrit (erythropoietin; Ortho Biotech), Epogen (erythropoietin; Amgen), Arnesp (erytropoietin). Combination compositions and combination administration methods of the present disclosure may also involve "whole cell" and "adoptive" immunotherapy methods. For instance, such methods may comprise infusion or re-infusion of immune system cells (for instance tumor-infiltrating lymphocytes (TILs), such as CC2+ and/or CD8+ T cells (for instance T cells expanded with tumor-specific antigens and/or genetic enhancements), antibody-expressing B cells or other antibody-producing or -presenting cells, dendritic cells (e.g., dendritic cells cultured with a DC-expanding agent such as GM-CSF and/or Flt3-L, and/or tumor-associated antigen-loaded dendritic cells), anti-tumor NK cells, so-called hybrid cells, or combinations thereof. Cell lysates may also be useful in such methods and compositions. Cellular "vaccines" in clinical trials that may be useful in such aspects include Canvaxin™, APC-8015 (Dendreon), HSPPC-96 (Antigenics), and Melacine® cell lysates. Antigens shed from cancer cells, and mixtures thereof (see for instance Bystryn et al., Clinical Cancer Research Vol. 7, 1882-1887, July 2001), optionally admixed with adjuvants such as alum, may also be components in such methods and combination compositions.

In some aspects of the disclosures, the antibody of the present disclosure is used in combination with another immune checkpoint inhibitor. As used herein, the term "immune checkpoint inhibitor" has its general meaning in the art and refers to any compound inhibiting the function of an immune inhibitory checkpoint protein. Inhibition includes reduction of function and full blockade. Preferred immune checkpoint inhibitors are antibodies that specifically recognize immune checkpoint proteins. A number of immune checkpoint inhibitors are known and in analogy of these known immune checkpoint protein inhibitors, alternative immune checkpoint inhibitors may be developed in the (near) future. In some aspects of the disclosures, the immune checkpoint inhibitor is an antibody selected from the group consisting of anti-CTLA4 antibodies, anti-PDl antibodies, anti-PDLl antibodies, anti-TIM-3 antibodies, anti-LAG3 antibodies, anti-B7H3 antibodies, anti-B7H4 antibodies, and anti-B7H6 antibodies. Examples of anti-CTLA-4 antibodies are described in US Patent Nos: 5,811,097; 5,811,097; 5,855,887; 6,051,227; 6,207,157; 6,682,736; 6,984,720; and 7,605,238. One anti-CDLA-4 antibody is tremelimumab, (ticilimumab, CP-675,206). In some aspects of the disclosures, the anti-CTLA-4 antibody is ipilimumab (also known as 10D1, MDX-D010) a fully human monoclonal IgG antibody that binds to CTLA-4. Examples of PD-1 and PD-L1 antibodies are described in US Patent Nos. 7,488,802; 7,943,743; 8,008,449; 8,168,757; 8,217,149, and PCT Published Patent Application Nos: WO03042402, WO2008156712, WO2010089411, WO2010036959, WO2011066342, WO2011159877, WO2011082400, and WO2011161699. In some aspects of the disclosures, the PD-1 blockers include anti-PD-Ll antibodies. In certain other aspects of the disclosures the PD-1 blockers include anti-PD-1 antibodies and similar binding proteins such as nivolumab (MDX 1106, BMS 936558, ONO 4538), a fully human IgG4 antibody that binds to and blocks the activation of PD-1 by its ligands PD-L1 and PD-L2; lambrolizumab (MK-3475 or SCH 900475), a humanized monoclonal IgG4 antibody against PD-1 ; CT-011 a humanized antibody that binds PD-1 ; AMP-224 is a fusion protein of B7-DC; an antibody Fc portion; BMS-936559 (MDX- 1105-01) for PD-L1 (B7-H1) blockade. Other immune-checkpoint inhibitors include lymphocyte activation gene-3 (LAG-3) inhibitors, such as IMP321, a soluble Ig fusion protein (Brignone et al., 2007, J. Immunol. 179:4202-4211). Other immune-checkpoint inhibitors include B7 inhibitors, such as B7-H3 and B7-H4 inhibitors. In particular, the anti-B7-H3 antibody MGA271 (Loo et al., 2012, Clin. Cancer Res. July 15 (18) 3834). Also included are TIM3 (T-cell immunoglobulin domain and mucin domain 3) inhibitors (Fourcade et al., 2010, J. Exp. Med. 207:2175-86 and Sakuishi et al., 2010, J. Exp. Med. 207:2187-94). As used herein, the term "TIM-3" has its general meaning in the art and refers to T cell immunoglobulin and mucin domain-containing molecule 3. The natural ligand of TIM-3 is galectin 9 (Gal9). Accordingly, the term "TIM-3 inhibitor" as used herein refers to a compound, substance or composition that can inhibit the function of TIM-3. For example, the inhibitor can inhibit the expression or activity of TIM-3, modulate or block the TIM-3 signaling pathway and/or block the binding of TIM-3 to galectin-9. Antibodies having specificity for TIM-3 are well known in the art and typically those described in WO2011155607, WO2013006490 and WO2010117057. In some aspects of the disclosures, the immune checkpoint inhibitor is an IDO inhibitor. Examples of IDO inhibitors are described in WO 2014150677. Examples of IDO inhibitors include without limitation 1-methyl-tryptophan (IMT), β- (3-benzofuranyl)-alanine, β-(3-benzo(b)thienyl)-alanine), 6-nitro-tryptophan, 6-fluoro-tryptophan, 4-methyl-tryptophan, 5-methyl tryptophan, 6-methyl-tryptophan, 5-methoxy-tryptophan, 5-hydroxy-tryptophan, indole 3-carbinol, 3,3'- diindolylmethane, epigallocatechin gallate, 5-Br-4-Cl-indoxyl 1,3-diacetate, 9- vinylcarbazole, acemetacin, 5-bromo-tryptophan, 5-bromoindoxyl diacetate, 3- Amino-naphtoic acid, pyrrolidine dithiocarbamate, 4-phenylimidazole a brassinin derivative, a thiohydantoin derivative, a β-carboline derivative or a brassilexin derivative. Preferably the IDO inhibitor is selected from 1-methyl-tryptophan, β-(3-benzofuranyl)-alanine, 6-nitro-L-tryptophan, 3-Amino-naphtoic acid and β-[3-benzo(b)thienyl] -alanine or a derivative or prodrug thereof.

In some aspects of the disclosures, the antibody of the present disclosure is used in combination with radiotherapy. Radiotherapy may comprise radiation or associated administration of radiopharmaceuticals to a patient. The source of radiation may be either external or internal to the patient being treated (radiation treatment may, for example, be in the form of external beam radiation therapy (EBRT) or brachytherapy (BT)). Radioactive elements that may be used in practicing such methods include, e.g., radium, cesium-137, iridium-192, americium-241, gold-198, cobalt-57, copper-67, technetium-99, iodide-123, iodide-131, and indium-Ill.

As used herein, the term "therapeutically effective amount" refers to an amount effective, at dosages and for periods of time necessary, to achieve a desired therapeutic result. A therapeutically effective amount of the antibody of the present disclosure may vary according to factors such as the disease state, age, sex, and weight of the individual, and the ability of the antibody of the present disclosure to elicit a desired response in the individual. A therapeutically effective amount is also one in which any toxic or detrimental effects of the antibody or antibody portion are outweighed by the therapeutically beneficial effects. The efficient dosages and dosage regimens for the antibody of the present disclosure depend on the disease or condition to be treated and may be determined by the persons skilled in the art. A physician having ordinary skill in the art may readily determine and prescribe the effective amount of the pharmaceutical composition required. For example, the physician could start doses of the antibody of the present disclosure employed in the pharmaceutical composition at levels lower than that required in order to achieve the desired therapeutic effect and gradually increase the dosage until the desired effect is achieved. In general, a suitable dose of a composition of the present disclosure will be that amount of the compound which is the lowest dose effective to produce a therapeutic effect according to a particular dosage regimen. Such an effective dose will generally depend upon the factors described above. For example, a therapeutically effective amount for therapeutic use may be measured by its ability to stabilize the progression of disease. Typically, the ability of a compound to inhibit cancer may, for example, be evaluated in an animal model system predictive of efficacy in human tumors. Alternatively, this property of a composition may be evaluated by examining the ability of the compound to induce cytotoxicity by in vitro assays known to the skilled practitioner. A therapeutically effective amount of a therapeutic compound may decrease tumor size, or otherwise ameliorate symptoms in a subject. One of ordinary skill in the art would be able to determine such amounts based on such factors as the subject's size, the severity of the subject's symptoms, and the particular composition or route of administration selected. An exemplary, non-limiting range for a therapeutically effective amount of an antibody of the present disclosure is about 0.1-100 mg/kg, such as about 0.1-50 mg/kg, for example about 0.1-20 mg/kg, such as about 0.1-10 mg/kg, for instance about 0.5, about such as 0.3, about 1, about 3 mg/kg, about 5 mg/kg or about 8 mg/kg. An exemplary, non-limiting range for a therapeutically effective amount of an antibody of the present disclosure is 0.02-100 mg/kg, such as about 0.02-30 mg/kg, such as about 0.05-10 mg/kg or 0.1-3 mg/kg, for example about 0.5-2 mg/kg. Administration may e.g. be intravenous, intramuscular, intraperitoneal, or subcutaneous, and for instance administered proximal to the site of the target. Dosage regimens in the above methods of treatment and uses are adjusted to provide the optimum desired response (e.g., a therapeutic response). For example, a single bolus may be administered, several divided doses may be administered over time or the dose may be proportionally reduced or increased as indicated by the exigencies of the therapeutic situation. In some aspects of the disclosures, the efficacy of the treatment is monitored during the therapy, e.g. at predefined points in time. In some aspects of the disclosures, the efficacy may be monitored by visualization of the disease area, or by other diagnostic methods described further herein, e.g. by performing one or more PET-CT scans, for example using a labeled antibody of the present disclosure, fragment or mini-antibody derived from the antibody of the present disclosure. If desired, an effective daily dose of a pharmaceutical composition may be administered as two, three, four, five, six or more sub-doses administered separately at appropriate intervals throughout the day, optionally, in unit dosage forms. In some aspects of the disclosures, the human monoclonal antibodies of the present disclosure are administered by slow continuous infusion over a long period, such as more than 24 hours, in order to minimize any unwanted side effects. An effective dose of an antibody of the present disclosure may also be administered using a weekly, biweekly or triweekly dosing period. The dosing period may be restricted to, e.g., 8 weeks, 12 weeks or until clinical progression has been established. As non-limiting examples, treatment according to the present disclosure may be provided as a daily dosage of an antibody of the present disclosure in an amount of about 0.1-100 mg/kg, such as 0.2, 0.5, 0.9, 1.0, 1.1, 1.5, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 45, 50, 60, 70, 80, 90 or 100 mg/kg, per day, on at least one of days 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40, or alternatively, at least one of weeks 1, 2, 3, 4, 5, 6, 7, 8, 9, 10, 11, 12, 13, 14, 15, 16, 17, 18, 19 or 20 after initiation of treatment, or any combination thereof, using single or divided doses every 24, 12, 8, 6, 4, or 2 hours, or any combination thereof.

Typically, the antibody of the present disclosure is administered to the subject in the form of a pharmaceutical composition which comprises a pharmaceutically acceptable carrier. Pharmaceutically acceptable carriers that may be used in these compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene- block polymers, polyethylene glycol and wool fat. For use in administration to a patient, the composition will be formulated for administration to the patient. The compositions of the present disclosure may be administered orally, parenterally, by inhalation spray, topically, rectally, nasally, buccally, vaginally or via an implanted reservoir. The used herein includes subcutaneous, intravenous, intramuscular, intra-articular, intra-synovial, intrasternal, intrathecal, intrahepatic, intralesional and intracranial injection or infusion techniques. Sterile injectable forms of the compositions of this disclosure may be aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono-or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation. The compositions of this disclosure may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include, e.g., lactose. When aqueous suspensions are required for oral use, the active ingredient is combined with emulsifying and suspending agents. If desired, certain sweetening, flavoring or coloring agents may also be added. Alternatively, the compositions of this disclosure may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols. The compositions of this disclosure may also be administered topically, especially when the target of treatment includes areas or organs readily accessible by topical application, including diseases of the eye, the skin, or the lower intestinal tract. Suitable topical formulations are readily prepared for each of these areas or organs. For topical applications, the compositions may be formulated in a suitable ointment containing the active component suspended or dissolved in one or more carriers. Carriers for topical administration of the compounds of this disclosure include, but are not limited to, mineral oil, liquid petrolatum, white petrolatum, propylene glycol, polyoxyethylene, polyoxypropylene compound, emulsifying wax and water. Alternatively, the compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water. Topical application for the lower intestinal tract can be effected in a rectal suppository formulation (see above) or in a suitable enema formulation. Patches may also be used. The compositions of this disclosure may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents. For example, an antibody present in a pharmaceutical composition of this disclosure can be supplied at a concentration of 10 mg/mL in either 100 mg (10 mL) or 500 mg (50 mL) single-use vials. The product is formulated for IV administration in 9.0 mg/mL sodium chloride, 7.35 mg/mL sodium citrate dihydrate, 0.7 mg/mL polysorbate 80, and Sterile Water for Injection. The pH is adjusted to 6.5. An exemplary suitable dosage range for an antibody in a pharmaceutical composition of this disclosure may between about 1 mg/m² and 500 mg/m². However, it will be appreciated that these schedules are exemplary and that an optimal schedule and regimen can be adapted taking into account the affinity and tolerability of the particular antibody in the pharmaceutical composition that must be determined in clinical trials. A pharmaceutical composition of the disclosure for injection (e.g., intramuscular, i.v.) could be prepared to contain sterile buffered water (e.g. 1 ml for intramuscular), and between about 1 ng to about 100 mg, e.g. about 50 ng to about 30 mg or more preferably, about 5 mg to about 25 mg, of an anti-BTLA antibody of the disclosure.

The disclosure will be further illustrated by the following figures and examples. However, these examples and figures should not be interpreted in any way as limiting the scope of the present disclosure.

### FIGURES:

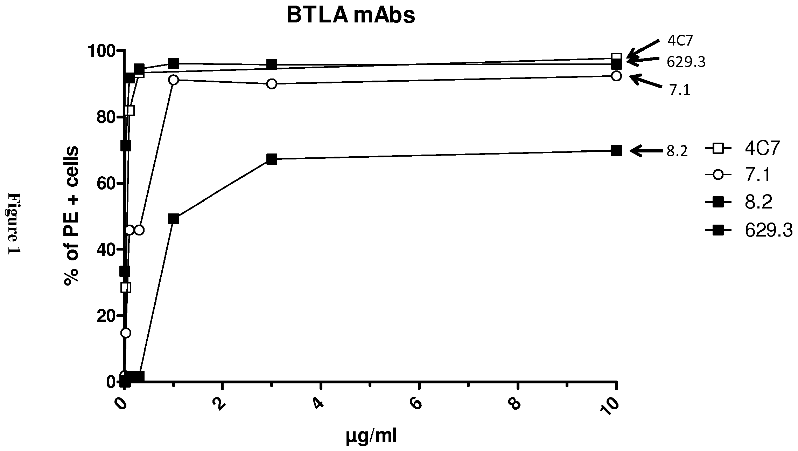

**Figure 1****: Binding of BTLA mAbs on PBMC.** PBMC were stained with purified anti-human BTLA mAbs for 20min at 4°C. After 2 washes, cells were incubated with a secondary anti-mouse IgG Pe (or an anti human IgG4 Pe for 4C7 humanized mAbs) for 20min at 4°C, CD3 (BD) and CD56 antibodies (Miltenyi). Cells were acquired on a FACSCanto II (BD) and analyzed with FlowJo (TreeStar). CD56+ cells were used as a negative control, and CD3+ cells were analyzed.
**Figure 2****: HVEM-BTLA inhibition of BTLA mAbs.** COS cells were transfected with 7,25µg of human BTLA in p3xFlag-myc-CMV25 (Sigma Aldrich) with XtremeGene9 (Roche) for 30min at RT. As negative control, COS were transfected with an empty p3xFlag-myc-CMV25. 24h later, cells were harvested and incubated with anti-BTLA (4C7, 8.2, 7.2 and 629.3) mAbs at various concentration (from 0,01µg/ml to 30 µg/ml) 30min at 4°C. After 2 washes, cells were incubated with 5µg/ml of HVEM-Fc at 4°C for 1 h. A anti-human IgG Fc gamma PE (Beckman Coulter) was then added for 20min at 4°C. Cells were acquired on a FACSCantoII (BD).
**Figure 3****: 629.****3** **and 4C7 do not cross bind.** L-BTLA (fibroblasts stably transfected with human BTLA) were incubated with increased concentration of either 4C7 mAb (top) or 629.3 mAb (bottom) from 0,001µg/ml up to 3 µg/ml for 30 min at 4°C. Cells were then incubated with anti-BTLA (4C7, 7.1, 8.2 and 629.3) mAbs coupled with Alexa-647 with a non-saturating dose for 20min at 4°C. LTK (non transfected fibroblasts) were used a as negative control. Cells were then fixed in formaldehyde 2% (Sigma Aldrich) and acquired on a FACS Fortessa (BD) and data were analyzed on FlowJo (TreeStar).
**Figure 4****: 629.****3** **increases Vγ9Vδ2 T cell proliferation.** Fresh PBMC were obtained from healthy blood at EFS and purified with Ficoll isolation (Eurobio). γδT cells were positively sorted with TCRγ/δ+ T Cell Isolation Kit from Miltenyi with the Automacs Pro (Miltenyi). Sorted γδT cells were stained with Cell Trace Violet (Life Technologies) according to manufacturer's protocol.γδT were then plated in 96-well round bottom at 50,000 cells per well in RPMI 10% FCS (Eurobio), 200 UI/ml of IL-2 (Proleukine) and 1µM of Zoledronic acid (Sigma) and with various anti-BTLA mAbs at 10µg/ml, IgG1 (Beckman Coulter) at 10µg/ml, or HVEM 18-10 (homemade) at 20µg/ml. Cells were incubated 5 days at 37°C, 5% CO2. γδT were harvested and stained for TCRγ/δ FITC (Beckman Coulter) and 7AAD (BD) to assess the proliferation of live cells. Cells were then acquired on a FACS Fortessa (BD) and data were analyzed on FlowJo10 (TreeStar). Only live γ/δT cells were analyzed.

### EXAMPLE:

The new mouse 629.3 mAb mAb having specificity for BTLA was generated and characterized in comparison with different BTLA antibodies, in particular the 7.1 and 8.2 mAbs disclosed in WO2010106051, and the 4C7 mAb disclosed in WO2011014438. T cells (CD3+) among PBMC were stained for BTLA with the anti BTLA mAbs at various concentration (from 0,01µg/ml to 10µg/ml). All the antibodies tested were able to recognize and bind BTLA onto the PBMC, but 629.3 mAb seems to have a higher binding to BTLA than the others mAbs, especially at a low dose (Figure 1). Then COS transfected with human BTLA were incubated with the anti BTLA mAbs and then with HVEM-Fc before staining with an anti human IgG Fc PE. The percentage of inhibition was determined as 100 - maximum of percentage of stained cells. All antibodies except 629.3 were able to block the interaction between HVEM Fc protein and BTLA (onto the cells) in a similar way (Figure 2). L-BTLA cells were then incubated with 4C7 or 629.3 mAbs and then with the Alexa-647 conjugated mAbs (4C7, 7.1, 8.2; 629.3). The median fluorescence intensity of cells was determined. 629.3 mAb and 4C7 mAb do not cross bind as we observe on Figure 3. On the other hand, 7.1 and 8.2 antibodies were not able to compete against 4C7 mAb at a high dose (Figure 3). We can conclude that 629.3 mAb does not cross bind with all the other mAbs tested (4C7, 7.1 and 8.2) and binds a different epitope than those antibodies. Finally, γδT were obtained after a positive sorting of fresh purified PBMC and stained for Cell Trace Violet to follow the proliferation. Cells were incubated 5 days in IL2/Zometa with the different mAbs prior to analysis by flow cytometry. The addition of antibodies in the culture of the γδT lead to an upregulation of their proliferation. Cells incubated with the HVEM 18.10 mAb disclosed in WO2014184360 is able to proliferate more than cells incubated with the mock control (IgG1) (Figure 4). In the same manner, we observe than all the anti-BTLA antibodies, including the 629.3 mAb were able to induce an increase of the γδT proliferation (Figure 4).

***In conclusion, we generated a new anti-BTLA antibody that does not inhibit the BTLA*/*HVEM interaction but still surprisingly inhibits the activation of BTLA.***

### Screening method of an antibody binding to the conformational epitope of SEQ ID NO:5 and SEQ ID NO:6

To screen an antibody which binds to the conformational epitope of SEQ ID NO:5 and SEQ ID NO:6, TK cells transfected with human BTLA are stained with saturing concentration (10 µg/ml) of 629.3 BTLA mAb during 30 minutes at 4°c. After 2 washes, different doses of other BTLA mAbs are tested (30 min at 4°C) for their competitive potential with 629.3 clone. Only mAbs that do not compete with 629.3 for the same binding site will still be able to recognize BTLA. Data are expressed as mean fluorescence intensity.

## Claims

1. An antibody having specificity for B and T lymphocyte attenuator (BTLA) **characterized in that**
i. it does not block the binding of HVEM to BTLA, and
ii. it increases the proliferation of Vγ9VδT cells
and wherein said antibody is either
i. an antibody having a heavy chain comprising the H-CDR1 as defined by the sequence ranging from the amino acid residue at position 31 to the amino acid residue at position 35 in SEQ ID NO:1; the H-CDR2 as defined by the sequence ranging from the amino acid residue at position 50 to the amino acid residue at position 65 in SEQ ID NO:1; the H-CDR3 as defined by the sequence ranging from the amino acid residue at position 98 to the amino acid residue at position 109 in SEQ ID NO:1; the L-CDR1 as defined by the sequence ranging from the amino acid residue at position 24 to the amino acid residue at position 40 in SEQ ID NO:2; the L-CDR2 as defined by the sequence ranging from the amino acid residue at position 56 to the amino acid residue at position 62 in SEQ ID NO:2; and, the L-CDR3 as defined by the sequence ranging from the amino acid residue at position 95 to the amino acid residue at position 102 in SEQ ID NO:2; or,
ii. a functional variant of a parent antibody as defined in (i) having CDR variant sequences which differ from corresponding CDR sequences of said parent antibody from only 1 substitution by conservative amino acid residue replacement.

2. The antibody of claim 1, which is an antibody comprising a heavy chain wherein the VH region has at least 70% of identity with SEQ ID NO:1 and a light chain wherein the VL region has at least 70% of identity with SEQ ID NO:2.

3. The antibody of any one of the preceding claims which is an antibody comprising a heavy chain wherein the VH region is identical to SEQ ID NO:1 and a light chain wherein the VL region is identical to SEQ ID NO:2.

4. A nucleic acid molecule which encodes a heavy chain and/or a light chain of the antibody of any one of Claims 1-3.

5. A host cell comprising the nucleic acid of claim 3.

6. The antibody of any one of Claims 1-3 for use as a medicament.

7. The antibody of any one of Claims 1-3, for use in treating a cancer.

8. The antibody for use according to Claim 7, wherein the cancer is a haematological malignancy, such as a lymphoma.

9. A pharmaceutical composition comprising the antibody of any one of Claims 1-3.

## Patentansprüche

1. Antikörper mit Spezifität für B- und T-Lymphozyten-Attenuator (BTLA), **dadurch gekennzeichnet, daß**
i. es blockiert nicht die Bindung von HVEM an BTLA, und
ii. es erhöht die Proliferation von Vγ9VδT-Zellen
und wobei der Antikörper entweder
i. Ein Antikörper mit einer schweren Kette beinhaltend die H-CDR1, definiert durch die Sequenz, die von dem Aminosäurerest an Position 31 bis zu dem Aminosäurerest an Position 35 in SEQ ID NO:1 reicht; die H-CDR2, definiert durch die Sequenz, die von dem Aminosäurerest an Position 50 bis zu dem Aminosäurerest an Position 65 in SEQ ID NO:1 reicht; die H-CDR3, definiert durch die Sequenz, die von dem Aminosäurerest an Position 98 bis zu dem Aminosäurerest an Position 109 in SEQ ID NO:1 reicht; die L-CDR1, definiert durch die Sequenz, die von dem Aminosäurerest an Position 24 bis zu dem Aminosäurerest an Position 40 in SEQ ID NO:2 reicht; die L-CDR2, definiert durch die Sequenz, die von dem Aminosäurerest an Position 56 bis zu dem Aminosäurerest an Position 62 in SEQ ID NO:2 reicht; und die L-CDR3, definiert durch die Sequenz, die von dem Aminosäurerest an Position 95 bis zu dem Aminosäurerest an Position 102 in SEQ ID NO:2 reicht; oder,
ii. eine funktionelle Variante eines Eltern-Antikörpers, wie in (i) definiert, mit CDR-Varianten-Sequenzen, die sich von entsprechenden CDR-Sequenzen des Eltern-Antikörpers nur durch eine Substitution durch konservativen Aminosäurerest-Ersatz unterscheiden.

2. Der Antikörper nach Anspruch 1, bei dem es sich um einen Antikörper handelt, der eine schwere Kette umfaßt, bei der die VH-Region zu mindestens 70% mit SEQ ID NO:1 identisch ist, und eine leichte Kette, bei der die VL-Region zu mindestens 70% mit SEQ ID NO:2 identisch ist.

3. Der Antikörper nach einem der vorhergehenden Ansprüche, der ein Antikörper ist, der eine schwere Kette umfasst, wobei die VH-Region identisch mit SEQ ID NO:1 ist, und eine leichte Kette, wobei die VL-Region identisch mit SEQ ID NO:2 ist.

4. Ein Nukleinsäuremolekül, das für eine schwere Kette und/oder eine leichte Kette des Antikörpers nach einem der Ansprüche 1-3 kodiert.

5. Eine Wirtszelle, umfassend die Nukleinsäure nach Anspruch 3.

6. Der Antikörper nach einem der Ansprüche 1 bis 3 zur Verwendung als Medikament.

7. Der Antikörper nach einem der Ansprüche 1 bis 3 zur Verwendung bei der Behandlung eines Krebses.

8. Antikörper zur Verwendung nach Anspruch 7, wobei der Krebs ein hämatologisches Malignom, wie z. B. ein Lymphom, ist.

9. Pharmazeutische Zusammensetzung, umfassend den Antikörper nach einem der Ansprüche 1-3.

## Revendications

1. Anticorps ayant une spécificité pour l'atténuateur des lymphocytes B et T (BTLA) **caractérisé en ce que**
i. il ne bloque pas la liaison de HVEM à BTLA, et
ii. il augmente la prolifération des cellules Vγ9VδT
et dans lequel ledit anticorps est
i. un anticorps ayant une chaîne lourde comprenant la H-CDR1 telle que définie par la séquence allant du résidu d'acide aminé en position 31 au résidu d'acide aminé en position 35 dans SEQ ID NO : 1 ; la H-CDR2 telle que définie par la séquence allant du résidu d'acide aminé en position 50 au résidu d'acide aminé en position 65 dans SEQ ID NO : 1 ; la H-CDR3 telle que définie par la séquence allant du résidu d'acide aminé en position 98 au résidu d'acide aminé en position 109 dans SEQ ID NO : 1 ; la L-CDR1 telle que définie par la séquence allant du résidu d'acide aminé en position 24 au résidu d'acide aminé en position 40 dans SEQ ID NO : 2 ; la L-CDR2 telle que définie par la séquence allant du résidu d'acide aminé en position 56 au résidu d'acide aminé en position 62 dans SEQ ID NO : 2 ; et, la L-CDR3 telle que définie par la séquence allant du résidu d'acide aminé en position 95 au résidu d'acide aminé en position 102 dans SEQ ID NO : 2 ; ou,
ii. un variant fonctionnel d'un anticorps parent tel que défini dans (i) ayant des séquences de variant de CDR qui diffèrent des séquences de CDR correspondantes dudit anticorps parent de seulement 1 substitution par un remplacement conservatif de résidu d'acide aminé.

2. Anticorps selon la revendication 1, qui est un anticorps comprenant une chaîne lourde dans lequel la région VH présente au moins 70 % d'identité avec SEQ ID NO : 1 et une chaîne légère dans lequel la région VL présente au moins 70 % d'identité avec SEQ ID NO : 2.

3. Anticorps selon l'une quelconque des revendications précédentes qui est un anticorps comprenant une chaîne lourde dans lequel la région VH est identique à SEQ ID NO : 1 et une chaîne légère dans lequel la région VL est identique à SEQ ID NO : 2.

4. Molécule d'acide nucléique qui code pour une chaîne lourde et/ou une chaîne légère de l'anticorps selon l'une quelconque des revendications 1 à 3.

5. Cellule hôte comprenant l'acide nucléique selon la revendication 3.

6. Anticorps selon l'une quelconque des revendications 1 à 3 pour une utilisation comme médicament.

7. Anticorps selon l'une quelconque des revendications 1 à 3 pour une utilisation dans le traitement d'un cancer.

8. Anticorps pour une utilisation selon la revendication 7, dans lequel le cancer est une malignité hématologique, telle qu'un lymphome.

9. Composition pharmaceutique comprenant l'anticorps selon l'une quelconque des revendications 1 à 3.
